# EUROPEAN PATENT APPLICATION

(11) **EP 2 042 177 A1**
(43) Date of publication of application: **01.04.2009**
(21) Application number: 08150834.3
(22) Date of filing: 30.01.2008
(51) Int. Cl.: A61K 31/485, A61K 45/06, A61P 1/12

(54) **Use of a combination of an opioid agonist and an opioid antagonist for the treatment of Crohn's disease and the symptoms associated with Crohn's disease**

(30) Priority: 26.09.2007 EP 07117241
(71) Applicant: EURO-CELTIQUE S.A., 1653 Luxembourg (LU)
(72) Inventor: Bennett-Kerr, Jo, Huntingdon Camps PE29 6 GE (GB)
(74) Representative: Bühler, Dirk

(57) **Abstract**

The present invention is concerned with the use of combinations of opioid agonists and antagonists for treating Crohn's disease.

## Description

The invention relates to the field of treating disorders of the gastrointestinal system in humans and animals.

### BACKGROUND OF THE INVENTION

Crohn's disease represents one of the two major types of inflammatory-bowel disease ("IBD"), in which the bowel becomes inflamed, often causing recurring abdominal cramps and diarrhea. The second major type of IBD is ulcerative colitis.

Crohn's disease, which can include regional enteritis, granulomatous ileitis, and ileocolitis, is characterised by a chronic inflammation of the intestinal wall. Most cases of Crohn's disease begin before age 30 and the majority start between the ages of 14 and 24.

Generally the disease affects the lowest portion of the small intestine (ileum) and the large intestine, but can occur in any part of the digestive tract. Typically, the full thickness of the intestinal wall is affected.

Early symptoms of Crohn's disease are chronic diarrhea, crampy abdominal pain, fever, loss of appetite, and weight loss. Complications associated with Crohn's disease include the development of intestinal obstructions, abnormal connecting channels (fistulas), and abscesses. The risk of cancer of the large intestine is increased in people who have Crohn's disease.

Often Crohn's disease is associated with other disorders such as gallstones, inadequate absorption of nutrients, amyloidosis, arthritis, episcleritis, aphthous stomatitis, erythema nodosum, pyoderma gangrenosum, ankylosing spondylitis, sacroilitis, uveitis, and primary sclerosing cholangitis.

Cramps and diarrhea, symptoms associated with Crohn's disease, can be relieved by anticholinergic drugs such as, diphenoxylate or loperamide. Generally, the drugs are taken orally before a meal.

Broad-spectrum antibiotics are often administered to treat the inflammatory symptoms of Crohn's disease. Thus, the antibiotic metronidazole is often administered when the disease affects the large intestine or causes abscesses and fistulas around the anus. Long-term use of metronidazole, however, can damage nerves, resulting in pins-and-needles sensations in the arms and legs.

Sulfasalazine and chemically related drugs can suppress mild inflammation, especially in the large intestine. These drugs, however, are less effective in sudden, severe flare-ups.

Corticosteroids, such as prednisolone, reduce fever and diarrhea and relieve abdominal pain and tenderness. Long-term corticosteroid therapy can result in serious side effects such as high blood-sugar levels, increased risk of infection, osteoporosis, water retention, and fragility of the skin.

Drugs such as azathioprine and mercaptourine can compromise the immune system and are often used for Crohn's disease in patients that do not respond to other drugs. These drugs usually need 3 to 6 months before they produce benefits and can cause serious side effects such as allergy, pancreatitis, and low white-blood-cell count.

When Crohn's disease causes the intestine to be obstructed or when abscesses or fistulas do not heal, surgery can be necessary to remove diseased sections of the intestine. Surgery, however, does not cure the disease, and inflammation tends to recur where the intestine is rejoined. In almost half of the cases a second operation is needed. The Merck Manual of Medical Information 528-530 (R. Berkow ed., 1997).

There is thus a continuing need for dosage forms which can be used to treat Crohn's disease or at least alleviate some of its major symptoms.

### SUMMARY OF THE INVENTION

It is one objective of the present invention to provide a pharmaceutical dosage form wich can be used for treating Crohn's disease.

It is also an objective of the present invention to provide a pharmaceutical dosage form which can be used for treating symptoms associated with Crohn's disease.

Furthermore, it is an objective of the present invention to provide a pharmaceutical dosage form which can be used for treating pain as a major symptom associated with Crohn's disease.

It is another objective of the present invention to provide a method of treating Chron's disease and the symptoms associated with Crohn's disease.

These and other objectives of the present invention as they become apparent from the ensuing description are attained by the subject matter of the indepent claim. Some of the preferred embodiments of the invention are put forward in the dependent claims.

In one embodiment, the present invention relates to pharmaceutical dosage forms comprising at least one opioid agonist or a pharmaceutically acceptable salt thereof in combination with at least one opioid antagonist or a pharmaceutically acceptable salt for treating Crohn's disease and/or for treating symptoms associated with Chrohn's disease in humans or animals.

In one embodiment, the opioid agonist may be selected from the group comprising morphine, oxycodone, hydromorphone, oxymorphone, propoxyphene, nicomorphine, dihydrocodeine, diamorphine, papaveretum, codeine, ethylmorphine, phenylpiperidine, methadone, dextropropoxyphene, buprenorphine, pentazocin, tilidine, tramadol, hydrocodone and salts thereof.

The opioid antagonist may be selected from the group comprising naloxone, naltrexone, nalmefene, nalorphine, nalbuphin, naloxonazinene, methylnaltrexone, ketylcyclazocine, norbinaltorphimine, naltrindol, 6-β-naloxol, 6-β-naltrexol and salts thereof.

In one embodiment, the opioid agonist and opioid antagonist are present in the pharmaceutical dosage forms as the free base.

In one of the preferred embodiments the opioid agonist and/or the opioid antagonist are present in the pharmaceutical dosage forms in the form of their pharmaceutically acceptable salts, preferably as the hydrochloride, sulfate, bisulfate, tartrate, nitrate, citrate, bitatrate, phosphate, malate, maleate, hydrobromide, hydroiodide, fumarate or succinate salt.

In one of the preferred embodiments, the pharmaceutical dosage forms are so-called controlled release dosage forms whith sustained release dosage forms being particularly preferred. In one of these embodiments, the opioid agonist or its pharamaceutically acceptable salt and the opioid antagonist or its pharmcaceutically acceptable salt are released from the dosage form in a sustained manner.

In one embodiment, the sustained release properties are essentially provided by a matrix which may be a diffusion matrix, an erosive matrix or a matrix which combines the properties of a diffusion and erosion matrix.

In a preferred embodiment, a diffusion matrix can comprise at least one hydrophobic polymer component and/or at least one fatty alcohol as the components which essentially affect the release of the opioid agonist and antagonist or of their pharmaceutically accpetable salts. A hydrophobic polymer may be e.g. a hydrophobic cellulose ether and preferable ethylcellulose. As a fatty alcohol, one may use e.g. lauryl alcohol, myrestyl alcohol, stearyl alcohol, cetostearyl alcohol, ceryl aclcohol and/or cetyl alcohol.

Alternatively or additionally, the sustained release properties of the phamaceutical dosage form may be achieved by a coating. Such a coating can made from e.g. a polymer, preferably from ethylcellulose and/or an acrylic polymer resin.

In one embodiment of the invention, the pharmaceutical dosage form may comprise an immediate release phase of the opioid agonist or its pharmaceutically acceptable salt. This may also be the case if the pharmaceutical dosage form as a whole is to be considered as a controlled release and preferably a sustained release dosage form.

The dosage forms in accordance with the invention can be formulated e.g. as tablets, capsules, granulates and/or powders.

In a preferred embodiment, dosage forms according to the present invention provide a therapeutic efficacy for at least 8 hours, preferably for at least 12 hours or at least 24 hours. As such, they may be administered three times a day, twice a day or only once a day.

In one of its more preferred embodiments, the present invention relates to pharmaceutical dosage forms for treating Crohn's disease which comprise oxycodone or a pharmaceutically accepptable salt thereof and naloxone or apharamaceutically acceptable salt thereof. Particularly preferred pharmaceutically acceptable salts of oxycodone and naloxone are their hydrochloride salts.

Such dosage forms may comprise between 5 to 160 mg of oxycodone or a pharmaceutically acceptable sals thereof and 2.5 to 40 mg of naloxone or a pharmaceutically acceptable salt thereof.

Typically, these dosage forms comprise oxycodone and naloxone in a weight ratio of 5:1,4:1, 3:1 and preferably of 2:1 of oxycodone:naloxone.

In a particularly preferred embodiment, the dosage form may comprise 10 mg of oxycodone or a pharmaceutically acceptable salt thereof and 5 mg of naloxone or a pharmaceutically acceptable salt thereof, 20 mg of oxycodone or a pharmaceutically acceptable salt thereof and 10 mg of naloxone or a pharmaceutically acceptable salt thereof or 40 mg of oxycodone or a pharmaceutically acceptable salt thereof and 20 mag of naloxone or a pharmaceutically acceptable salt thereof.

The above-mentioned dosage forms comprising oxycodone and naloxone or their pharmaceutically acceptable salts may, of course, also be formulated as controlled and preferably as sustained release dosage forms. To this end, the dosage forms can be e.g. matrix-based and/or coating-based sustained release dosage forms as mentioned above.

In one of its preferred embodiments, such sustained release dosage forms release oxycodone or a pharmaceutcially acceptable salt thereof and naloxone or a pharmaceutically acceptable salt thereof by the following in vitro dissolution rates when measured according to the European Pharmacopoeia paddle test at 50 rpm in 900 ml 0.1 N HCl pH 1.2 using UV detection at 270 nm:
10 - 30 % by weight of oxycodone or said salt thereof at 15 min,
40 - 65 % by weight of oxycodone or said salt thereof at 2 h,
≥ 80% by weight of oxycodone or said salt thereof at 10 h,
10 - 30 % by weight of naloxone or said salt thereof at 15 min,
40 - 65 % by weight of naloxone or said salt thereof at 2 h,
≥ 80% by weight of naloxone or said salt thereof at 10 h.

Pharmaceutical dosage forms in accordance with the invention which comprise oxycodone and naloxone or their pharmaceutically acceptable salts provide preferably a therapeutic efficacy of at least twelve hours.

In another preferred embodiment, pharmaceutical dosage forms in accordance with the invention which comprise oxycodone and naloxone or their pharmaceutically acceptable salts provide a mean tₘₐₓ four oxycodone at about 1 to about 17 hours, at about 2 to about 15 hours, at about 3 to about 8 hours or at about 4 to about 5 hours after single dose administration to healthy human subjects.

Such dosage forms may also provide a mean AUCt value for oxycodone of about 100 ng•h/mL to about 600 ng•h/mL, about 400 ng•h/mL to about 550 ng•h/mL, or about 450 to about 510 ng•h/mL after single dose administration to healthy human subjects.

In yet another embodiment, such dosage forms provide a mean Cₘₐₓ for oxycodone of about 5 ng/mL to about 50 ng/mL, about 30 ng/mL to about 40 ng/mL or about 35 ng/mL after single dose administration to healthy human subjects.

The present invention in one its most preferred embodiments relates to a pharmaceutical dosage form for treating Crohn's disease wherein the pharmaceutical dosage form comprises oycodone hydrochloride and nalxone hydrochloride in a 2:1 weight ratio with oxycodone hydrochloride being present in an amount of 10 to 40 mg and naloxone hydrochloride being present in an amount of 5 to 20 mg, wherein the pharmaceutical dosage form is a sustained release dosage form which preferably is based on a sustained release diffusion matrix and wherein the sustained release dosage form releases oxycodone or a pharmaceutcially acceptable salt thereof and naloxone or a pharmaceutically acceptable salt thereof by the following in vitro dissolution rates when measured according to the European Pharmacopeia paddle test at 50 rpm in 900 ml 0.1 N HCl pH 1.2 using UV detection at 270 nm:
10 - 30 % by weight of oxycodone or said salt thereof at 15 min,
30 - 50 % by weight of oxycodone or said salt thereof at 1 h,
40 - 65 % by weight of oxycodone or said salt thereof at 2 h,
60 - 85 % by weight of oxycodone or said salt thereof at 4 h,
70 - 95 % by weight of oxycodone or said salt thereof at 7 h,
≥ 0% by weight of oxycodone or said salt thereof at 10 h,
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

The present invention also relates to a method of treating a human subject being afflicted by Crohn's disease by administering one of the above described dosage forms.

The present invention relates to the use of an opioid agonist or a pharmaceutically acceptable salt thereof and an opioid antagonist or a pharmaceutically acceptable salt thereof in the manufacture of a pharmaceutical dosage form for treating Crohn's disease in humans.

The expressions "treatment of Crohn's disease" or " treating Crohn's disease" should not be interpreted as being limited to the treatment of the disease itself only. They are used in the meaning to also inlcude the treatment of any symptoms, as e.g. pain such as crampy abdominal pain, episcleritis or uveitis, cramps, diarrhea such as chronic diarrhea, constipation, flatus, vomiting, nausea, itchiness, as well as systemic symptoms such as fever, growth retardation, loss of appetite, weight loss, which are associated with Crohn's disease. Furthermore, Crohn's disease may be associated with symptoms induced by rheumatologic diseases such as seronegative spondyloarthropathy, or with symptoms manifesting in skin, blood, and endocrine system's diseases. Also, it may be accompanied by neurological complications, such as stroke, myopathy, headache and depression. Also, the so-called "small bowel bacterial overgrowth syndrome" may be a symptom associated with Crohn's disease.

Thus, in one preferred embodiment, the present invention relates to pharmaceutical dosage forms comprising at least one opioid agonist or a pharmaceutically acceptable salt thereof in combination with at least one opioid antagonist or a pharmaceutically acceptable salt for treating symptoms associated with Crohn's disease in humans or animals.

In an even preferred embodiment, the present invention relates to pharmaceutical dosage forms comprising at least one opioid agonist or a pharmaceutically acceptable salt thereof in combination with at least one opioid antagonist or a pharmaceutically acceptable salt for treating pain as a major symptom associated with Crohn's disease in humans or animals.

The manufacture of above-described preferred pharmaceutical dosage forms is preferred.

### DETAILED DESCRIPTION OF THE INTENTION

The present invention is based on the finding that pharmaceutical compositions comprising an opioid agonist or a pharmaceutically acceptable salt thereof and an opioid antagonist or a pharmaceutically acceptable salt thereof can be used four treating Crohn's disease.

Before some of the embodiments of the present invention are described in further detail, the following definitions are introduced.

As used in this specification and the claims, the singular forms of "a" and "an" also include the respective plurals unless the context clearly dictates otherwise.

The terms "about" and "approximately" in the context of the present invention denote a level or interval of accuracy that a person skilled in the art will understand to still ensure the technical effect of the feature in question. For numerical values, the terms typically indicate a deviation of ± 10% and preferably of ± 5%.

It is to be understood that the term "comprising" is not limiting. However, for the purposes of the present invention the term "consisting of is considered to be a preferred embodiment of the term "comprising". If hereinafter a group is defined to comprise at least a certain number of embodiments, this is meant also to encompass a group, which preferably consists of these embodiments only.

As mentioned above, the present invention relates to a pharmaceutical preparation comprising an opioid agonist or a pharmaceutically acceptable salt thereof and an opioid antagonist or a pharmaceutically acceptable salt thereof for treating Crohn's disease in a human subject.

The present invention also relates to the use of an opioid agonist or a pharmaceutically acceptable salt thereof and an opioid antagonist or a pharmaceutically acceptable salt thereof in the manufacture of a pharmaceutical dosage form for treating Crohn's disease in humans or animals.

In a third aspect, the present invention relates to a method of treating Crohn's disease in a human subject by administering to said subject a pharmaceutical composition comprising an opioid agonist or a pharmaceutically acceptable salt thereof and an opioid antagonist or a pharmaceutically acceptable salt thereof.

In the context of the present invention, the term "opioid agonist" is used as known in the art. For the purposes of the present invention it will be considered to be equivalent to the term "opioid analgesic". Typically, a pharmaceutically active agent will be considered to be an opioid analgesic or opioid agonist if it belongs to Class NO2A of opioid analgesics according to the Anatomical Therapeutic Chemical classification (ATC classification) of the World Health Organisation (WHO). Preferably, an opioid agonist may be selected from the group comprising morphine, oxycodone, hydromorphone, propoxyphene, nicomorphine, dihydrocodeine, diamorphine, papaveretum, codeine, ethylmorphine, phenylpiperidine and derivates thereof, methadone, dextropropoxyphene, buprenorphine, pentazocine, tilidine, tramadol, hydrocodone. Further examples for useable analgesic according to the invention are meperidine, oxymorphone, alphaprodine, anileridine, dextromoramide, metopone, levorphanol, phenazocine, etoheptazine, propiram, profadol, phenampromide, thiambuten, pholcodeine, codeine, dihydrocodeinon, fentanyl, 3-trans-dimethylamino-4-phenyl-4-trans-carbethoxy-A'-cyclohexen, 3-dimethylamino-0-(4-methoxyphenyl-carbamoyl)-propiophenone oxims, (-)β-2'-hydroxy-2, 9-dimethyl-5-phenyl-6, 7-benzomorphane, (-)2'-hydroxy-2-(3-methyl-2-butenyl)-9-methyl-5-phenyl-6, 7-benzomorphane, pirinitramide, (-)α-5,9-diethyl-2' hydroxy-2-methyl-6, 7-benzomorphane, ethyl 1-(2-dimethylaminoethyl)-4,5,6,7-tetrahydro-3-methyl-1-4-oxo-6-phenyl-indol-2-carboxylate, 1-benzoylmethyl-2,3-dimethyl-3- (m-hydroxyphenyl) -piperidine, N-allyl-7α(1-R-hydroxy-1-methylbutyl)-6,14-endoethanotetrahydronororipavine, (-)2'-hydroxy-2-methyl-6,7-benzomorphane, noracylmethadol, phenoperidine, α-d1-methadol, α-1-methanol, β-d1-acetytmethadol, α-1-acetylmethadol and β-1-acetylmethadol. Preferred opioid agonists according to the present invention are oxycodone, hydrocodone, hydromorphone, morphine, codeine, dihydrocodeine, oxymorphone and fentanyl. The opioid agonist oxycodone can be Particularly preferred.

According to the present invention "opioid antagonists" comprise such compounds that counteract the effect of opioid agonists. Such compounds can also be found in the ATC classification of the WHO. Opioid antagonists in accordance with the preset invention may be selected from the group comprising naloxone, naltrexone, nalmefene, nalorphine, nalbuphine, naloxoneazinen, methylnaltrexone, ketylcyclazocine, norbinaltorphimine, naltrindol, 6-β-naloxol and 6-β-naltrexol. The opioid antagonist naloxone can be particularly preferred.

The opioid agonist and antagonist may be present in the pharmaceutical dosage forms of the present invention as the free base. However, an opioid agonist or antagonist may also be present in the form of its pharmaceutically acceptable salts. Such salts include e.g. the hydrochloride salt, the sulfate salt, the bisulfate salt, the tartrate salt, the nitrate salt, the citrate salt, the bitartrate salt, the phosphate salt, the malate salt, the maleate salt, the hydrobromide salt, the hydroiodide salt, the fumerate salt, the succinate salt and the like. The pharmaceutically active agents may also be present as base addition salts such as the metal salt of alkali metals including lithium, sodium and kalium. The pharmaceutically active compounds may, of course, also be present in the form of derivatives of the free base. Such derivatives include e.g. esters.

If oxycodone is used as an opioid agonist and naloxone is considered as an opioid antagonist, it can be preferred to use the hydrochloride salt thereof.

In one of the preferred embodiments of the invention, the pharmaceutical dosage forms may be formulated to ensure a controlled release of the opioid agonist and/or antagonist. Such dosage forms may therefore be designated as controlled release (CR) pharmaceutical dosage forms.

The term "controlled release dosage for" is typically used to highlight that a pharmaceutical dosage form is not an immediate release (IR) pharmaceutical dosage form. An oral immediate release pharmaceutical dosage form will typically release substantially all of the pharmaceutically active agents within a short time of administration. It is a commonly accepted definition in the art that an IR dosage form releases 70 by weight of the pharmaceutically active agents within 30 minutes after administration. The release is typically measured using the European Pharmacopeia paddle test as mentioned above.

A controlled release dosage form may designate a pharmaceutical dosage form that releases the active agent only after the dosage form has reached a certain site of the body, i.e. the stomach or the gastro-intestinal tract. Additionally or alternatively, it may designate a dosage form, which releases the active agent over a prolonged period of time. In the latter case, controlled release dosage forms are designated as sustained release dosage forms.

A site-specific controlled release of pharmaceutically active agent from a pharmaceutical dosage form may e.g. be achieved by that the release is made dependent on the pH value of the liquids that the dosage form encounters when passing through a human body. Such a pH-dependent release may allow that a dosage form releases the active agent not in the stomach but only in the gastro-intestinal tract.

The term "sustained release" refers to the release of pharmaceutically active compounds from a dosage form over an extended period of time, but not necessarily to the release at a defined place.

Of course, the above principles can be combined. For example, a pharmaceutical dosage form may comprise an enteric coating in order to ensure that the active agent is released only in the gastro-intestinal tract. The release during the gastro-intestinal passage may, however, display the characteristics of sustained release.

In general sustained release in the context of the present invention means that the opioid agonist and the opioid antagonist are released from the pharmaceutical dosage form over a time period of at least 2 hours. Of course, the release of the pharmaceutically active agents from the dosage form may take place over time periods of at least 4 hours, at least 6 hours, at least 10 hours, at least 12 hours or at least 14 hours. The release can be measured by the European Pharmacopoeia paddle test as mentioned above.

Reference to the European Pharmacopeia paddle test or the US Pharmacopeia Basket test in the context of *in vitro* release data refers only to the method of measurement but not any way of evaluating the measured data even if such evaluation approaches are mentioned in these Pharmacopoeias. Thus, the indicated release values do not relate to average values of e.g. six measurements unless indicated otherwise.

A preferred embodiment of the present invention relates to pharmaceutical dosage forms and their use as described above wherein the dosage forms provide for a sustained release of the opioid agonist and the opioid antagonist or the pharmaceutically acceptable salt thereof.

Sustained release characteristics can be achieved by different formulation approaches. For example, a pharmaceutical dosage form may comprise a sustained release matrix in which the pharmaceutically active agent is embedded in order to achieve the sustained release properties of the dosage form.

In another embodiment, a coating approach may be used to ensure the sustained release characteristics of a pharmaceutical dosage form.

These approaches for achieving sustained release of active agent, i.e. use of a matrix or use of a coating may of course also be combined. The person skilled in the art is further aware of other technical approaches for achieving a sustained release of the dosage form which include e.g. osmotically driven sustained release dosage form.

In a preferred embodiment, the present invention contemplates pharmaceutical dosage forms which comprise a matrix for achieving a sustained release of the agonist and/or the opioid antagonist or the pharmaceutically acceptable salt thereof. To this end, the matrix may comprise a substantial amount of the opioid agonist and antagonist that are present within the dosage form. Typically, if a matrix system is used, the pharmaceutically active agents such as the opioid agonist or opioid antagonist will be dispersed throughout a matrix-forming material.

The matrix-forming materials may be chosen to achieve an erosive matrix, a diffusion matrix or a matrix system which combines the characteristic of an erosive or and a diffusion matrix. Suitable materials for inclusion in a sustained release matrix include:
(a) Hydrophillic or hydrophobic polymers, such as gums, cellulose ethers, acrylic resins and protein derived materials. Of these polymers, the cellulose ethers, especially alkylcelluloses are preferred. The preparation may conveniently contain between 1% and 80% (by weight) of one or more hydrophilic or hydrophobic polymers.
(b) Digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils and waxes. Hydrocarbons having a melting point of between 25 and 90°C are preferred. Of these long chain hydrocarbon materials, fatty (aliphatic) alcohols are preferred.The preparation may conveniently contain up to 60% (by weight) of at least one digestible, long chain hydrocarbon.
(c) Polyalkylene glycols. The preparation may suitably contain up to 60% (by weight) of one or more polyalkylene glycols.

In a preferred embodiment, the pharmaceutical dosage forms as described in the present invention will use a diffusion matrix for achieving sustained release of the opioid agonist and antagonist from the pharmaceutical dosage form.

To this end, the diffusion matrix may be made from a hydrophobic polymer and/or a C₁₂-C₃₆ fatty alcohol.

As regards the hydrophobic polymer, use of a hydrophobic cellulose ether and particularly ethyl cellulose may be preferred.

As regards the fatty alcohol, use of lauryl, myristyl, stearyl, cetylstearyl, ceryl and/or cetylalcohol will be preferably considered. The use of stearyl alcohol is particularly preferred. A particularly preferred embodiment relates to pharmaceutical dosage forms in which the sustained release properties of the opioid agonist and opioid antagonist or the pharmaceutically acceptable salt thereof are provided by a diffusion matrix which is made from a hydrophobic polymer such as from ethyl cellulose and a fatty alcohol. The matrices of some of some of the preferred embodiments of the invention, which may e.g. be made from the aforementioned combination of ethyl cellulose and stearyl alcohol, will be substantially non-swellable diffusion matrix.

The term "substantially non-swellable diffusion matrix" indicates that the matrix will be substantially non-erosive, i.e. that the size of the matrix will not significantly increase upon contact with fluids. Typically, the volume of a substantially non-swellable diffusion matrix will increase at maximum up to 100 %, preferably at maximum up to 75 %, more preferably at maximum up to 50 %, even more preferably at maximum up to 25% and most preferably at maximum up to 10 % or at maximum up to 5 % in volume upon contacting an aqueous solution.

Pharmaceutical dosage forms which comprise a hydrophobic polymer with hydrophobic cellulose ethers such as ethyl cellulose being preferred as the sole or one of the components for providing a sustained release (non-swellable) diffusion matrix, will use an amount of such polymer of between 5 to 20%, preferably of between 6 and 15% by weight and more preferably of between 7 to 10% by weight. An amount of about 8% by weight can be particularly preferred. The percentages indicate the amount of the matrix-forming material with respect to the total weight of the pharmaceutical dosage form.

Pharmaceutical dosage forms, which comprise a fatty alcohol as the sole or one of the components for providing a sustained release diffusion matrix, will use an amount of fatty alcohol in the matrix of between 10 to 40%, preferably of between 15 to 35 % and more preferably of between 17 to 25% by weight. An amount of about 20% by weight can be particularly preferred. These percentages again indicate the amount of fatty alcohol based on the total weight of the dosage form.

If the pharmaceutical dosage form comprises a matrix as the release-influencing structural unit, the pharmaceutical dosage form may be substantially identical in term of form and size with the matrix.

The person skilled in the art is further aware that such a sustained release matrix may also contain other pharmaceutically acceptable ingredients and excipients which are conventional in the pharmaceutical art such as diluents, lubricants, fillers, binders, flowing agents, colourants, flavourants, surfactants, pH-adjusters, anti-tacking agents. These excipients will typically have no substantial impact on the overall release behaviour of the pharmaceutical dosage form.

Typical examples of fillers comprise lactose, glucose, saccharose, starch and their hydrol-sates, microcrystalline cellulose, calcium salts like calcium hydrogen phosphate etc. Granulating aids comprise *inter* alia povidone. Flowing agents and lubricant comprise *inter alia* highly dispersed silica, talcum, magnesium oxide and magnesium stearate. Matrix-based dosage form may e.g. comprise a cosmetic coating.

As mentioned above, sustained release characteristics of a pharmaceutical dosage form may also be achieved by a film coating that governs the release of the active agents from the dosage form. To this end, the pharmaceutical dosage form may comprise a carrier which is associated with the opioid agonist and/or opioid antagonist or the pharmaceutically acceptable salts thereof. For example, one may use nu-pareil beads, sugar beads etc. on and/or into which the pharmaceutically active agents are disposed. Such active-associated carriers may then be overcoated with a coating that provides sustained release characteristics. Suitable controlled release coating materials include. The person skilled in the art will be aware of other formulation principles that can be used to achieve sustained release characteristics.

It is to be understood that for the question of whether a pharmaceutical dosage form is to be considered as a sustained release dosage form, the release behaviour of the dosage form as a whole must be considered. this, a sustained release dosage form may comprise a matrix with the pharmaceutically active agent that provides a sustained release of the active agent on which a layer of active agent is disposed that will be immediately released after administration of the dosage form. Such immediate release phases may allow fast achievement of a therapeutic effect while the sustained release matrix will ensure that the active agents are released over a prolonged period of time so that the therapeutic effect is maintained over a time longer than it would be for a purely immediate release dosage form.

This illustrates that a dosage form may still be considered as providing sustained release characteristics if it comprises an immediate release phase.

In a preferred embodiment the present invention may not only provide a sustained release of the opioid agonist and/or opioid antagonist or the pharmaceutically acceptable salt thereof but also ensure that the active agents are released in an independent and invariant manner.

Independent release means that given the presence of at least two active compounds a change of the absolute amount of one compound does not influence the release profile of the other compound, the amount of which is not changed. This means that, for example, the release profile of oxycodone as it is observed for an oxycodone/naloxone combination with 10 mg oxycodone and 5 mg naloxone does not change substantially if a corresponding preparation with the same formulation contains 10 mg oxycodone and 10 mg of naloxone. The comparison will, of course, typically refer to a situation where preparations of substantially equal composition are compared with respect to their release profile. Dosage forms of substantially equal composition will have different amounts of active compounds but are otherwise basically the same with respect to the components of the composition, which essentially influence the release behaviour. If e.g. a preparation comprising 10 mg oxycodone and 5 mg naloxone is compared with a preparation comprising 10 mg oxycodone and 10 mg naloxone, both preparations, provided that they have the same total weight, will provide for substantially the same release profile for oxycodone if the difference in the naloxone amount is replaced by a component in the formulation that typically does not influence the release behaviour. Such a component may be a filler such as lactose. The release profile for one compound will be considered to be substantially the same if the release at a specified point in time differs by no more than 20%, preferably by no more than 15 %, more preferably by no more than 10 % and most preferably by now more than 5% between the two preparations. For this comparison one will consider the mean release values for that specific point in time as obtained by the measurement of six dosage forms for each strength considered.

Invariant release in the context of the present invention means that the release profile of an active agent will be substantially the same even if the absolute amount of that active agent within the pharmaceutical dosage form is changed. Thus, if for example the dosage form comprises 10 mg of oxycodone and 5 mg of naloxone, is compared with a dosage form comprising 20 mg of oxycodone and 5 mg of naloxone, the release of oxycodone will in both cases be substantially the same.

As for the issue of independent release this, of course, implies that compositions of substantially the same composition are compared meaning that any change in the amount of an active agent is replaced by components that essentially do not influence the release behaviour of the pharmaceutical dosage form. The release profile of the active agent, the amount of which is different between two compositions, will again be considered to be substantially the same if the release value at a defined point in time does not differ for the two compositions by more than 20%, preferably by no more than 15%, more preferably by no more than 10% and most preferably by no more than 5%. For this comparison one will consider the mean release values for that specific point in time as obtained by the measurement of six dosage forms for each strength considered.

The person skilled in the art is aware that dosage forms in accordance with the invention comprising an opioid agonist or antagonist or their pharmaceutically acceptable salts can be produced by different methods of manufacturing including stray granulation or extrusion. Production by extrusion may be preferred in the context of the present invention as it allows for a continuous process.

In one preferred embodiment, pharmaceutical preparations or preliminary stages thereof are produced by melt extrusion with co- or counter-rotating extruders comprising at least two screws. These extruders may also comprise kneading elements.

Generally, extrusion is an established production process in pharmaceutical technology and is known to the person skilled in the art. The person skied in the art is aware that during the extrusion process, various parameters, such as the feeding rate, the screw speed, the heating temperature of the different extruder zones (if available), the water content, etc. may be varied in order to produce products of the desired characteristics. The Example section provides for numerous examples of preparations according to the invention that have been produced by extrusion.

The aforementioned parameters will depend on the specific type of extruder used. During extrusion the temperature of the heating zones, in which the components of the inventive formulation melt, may be between about 40 to about 120 °C, preferably between about 50 to about 100 °C, more preferably between about 50 to about 90 °C and even more preferably between about 60 to about 80 °C, particularly if counter-rotating twin screw extruders (such as a Leistritz ZSE Micro 18 GGL) are used. The person skilled in the art is well aware that not every heating zone has to be heated. Particularly behind the feeder where the components are mixed, cooling at around 25 °C may be advisable. The temperature at the extruder's die may also be regulated and fall in the range of about 50 to about 80°C with about 60°C being a possibly preferred value. The bar at the noozle may be e.g. in the range of about 1 to 10 bar. The screw speed may vary between about 100 to about 500 revolutions per minute (rpm), preferably between about 100 to about 250 rpm and more preferably between about 100 to about 200 rpm, particularly if counter-rotating twin screw extruders (such as a Leistritz Micro 18 GGL) are used. The geometry and the diameter of the nozzle may be selected as required. The diameter of the nozzle of commonly used extruders typically is between 1 to 10 mm, preferably between 2 to 8 mm and most preferably between 3 to 5 mm. The different extruders may differ with respect to their set up. The ratio of length versus diameter of the screw of extruders that may be used for production of inventive preparations is typically around 40:1.

Generally, the temperatures of the heating zones have to be selected such that no temperatures develop that may destroy the pharmaceutically active compounds. The feeding rate und screw speed will be selected such that the pharmaceutically active compounds are released from the preparations produced by extrusion in a sustained, and preferably independent and invariant manner. If e.g. the feeding rate is increased, the screw speed may have to be increased correspondingly to ensure the same retardation.

In a particularly preferred embodiment, one uses a counter-rotating twin screw extruder without paddle means such as a Leistritz ZSE Micro 18 or Leistritz Micro 27 with a die plate and without kneading elements. For these embodiment, the feeding rate of the components used for the production of the formulation in accordance with the invention can be between about 1-3 kg/h, preferably between about 1-2 kg/h. The temperature within a first heating (feeding) zone may be about 20 to about 30°C. The temperature within a second heating zone may be about 50°C to about 60°C. The temperature of other heating zone the number of which can extend to eight zones may be between about 55° to about 85°C. The screw speed can be between about 1 to about 500 rpm. The diameter of the nozzle can be between about 1 to about 10 mm.

The production of formulations in accordance with the invention by extrusion is preferred with the formulations comprising opioid agonists and opioid antagonists. Particularly preferred is the production of formulations in accordance with the invention that comprise oxycodone and naloxone, wherein the preferred weight ratios of agonist to antagonist are within a weight ratio ranging from maximal 25:1, preferably of 20:1, 15:1, 10:1, 5:1 and 2:1. The ratio of 2:1 is particularly preferred.

A particularly preferred embodiment relates to pharmaceutical dosage forms and their use for treating Crohn's disease, which comprise oxycodone or a pharmaceutically acceptable salt thereof as the opioid agonist and naloxone or a pharmaceutically acceptable salt thereof as the opioid antagonist. The pharmaceutically acceptable salt may be selected from the above-mentioned list. However, the use of the hydrochloride salt may be particularly preferred.

The amount of oxycodone or the pharmaceutically acceptable salt thereof will typically vary between 5 to 160 mg, preferably between 10 to 80 mg, more preferably between 20 to 40 mg. The amount of naloxone will vary between 2.5 to 80 mg, preferably between 5 to 40 mg, more preferably between 10 and 20 mg.

The weight ratio between oxycodone and naloxone will be 5:1, preferably 4:1, more preferably 3:1 and particularly preferably 2:1.

Pharmaceutical dosage forms, which comprise oxycodone or the hydrochloride salt thereof and naloxone or the hydrochloride salt thereof in a 2:1 ratio are particularly preferred for treating Crohn's disease. Thus, these dosage forms may comprise 10 mg of oxycodone or oxycodone hydrochloride and 5 mg of naloxone, (or the corresponding amounts of the hydrochloride salts), 20 mg of oxycodone and 10 mg of naloxone (or the corresponding amounts of the hydrochloride salts) or 40 mg of oxycodone and 20 mg of naloxone (or the corresponding amounts of the hydrochloride salts). The dosage strength of 20 mg oxycodone and 10 mg of naloxone (or the corresponding amounts of the hydrochloride salts) can be particularly preferred in view of their appreciation by patients.

Of course, the pharmaceutical compositions in accordance with the invention which comprise oxycodone or a pharmaceutically acceptable salt thereof and naloxone or a pharmaceutically acceptable salt thereof then may be formulated to provide a sustained release behaviour as described above. In the context of oxycodone and naloxone combinations or pharmaceutically acceptable salts thereof with the above indicated amounts of the active agents and the above indicated weight ratios of oxycodone or a salt thereof and naloxone or a salt thereof, it can be particularly preferred to use a diffusion matrix for providing sustained release characteristics. Such a diffusion matrix may be preferably non-swellable and can be made as described above from e.g. hydrophobic polymers including hydrophobic cellulose ethers and preferably ethyl cellulose and/or fatty acids. The combination of e.g ethyl cellulose and at least one fatty alcohol as the matrix-forming components can be preferred.

If ethyl cellulose and fatty alcohols are used as the release influencing components of the sustained release matrix, they may be present in the pharmaceutical dosage form by the above mentioned amounts.

Even though dosage forms comprising a sustained release diffusion matrix of the above mentioned composition may be preferred, the invention certainly also considers pharmaceutical dosage forms comprising oxycodone and naloxone or salts thereof for which the sustained release characteristics are provided by a coating in the context of treating Crohn's disease. Other sustained release technologies are also to be considered.

The pharmaceutical dosage forms comprising oxycodone and naloxone or the salts thereof in the above mentioned amounts and ratios may preferably display the following *in vitro* dissolution rates when measured according to the European Pharmacopoeia paddle test at 50 rpm in 900 ml 0.1 N HCl pH 1.2 using UV detection at 270 nm:
10 - 30 % by weight of oxycodone or said salt thereof at 15 min,
30 - 50 % by weight of oxycodone or said salt thereof at 1 h,
45 - 65 % by weight of oxycodone or said salt thereof at 2 h,
60 - 80 % by weight of oxycodone or said salt thereof at 4 h,
75 - 95 % by weight of oxycodone or said salt thereof at 7 h,
≥ 80% by weight of oxycodone or said salt thereof at 10 h,
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 90 % by weight of naloxone or said salt thereof at 4 h,
75 - 95 % by weight of naloxone or said salt thereof at 7 h, and
≥ 80% by weight of naloxone or said salt thereof at 10 h.

These release values preferably apply if the dosage form comprises up to 20 mg of oxycodone, such as 10 mg of oxycodone and 5 mg of naloxone or 20 mg of oxycodone and 10 mg of naloxone (or the equivalent amounts of corresponding salts thereof).

The pharmaceutical dosage forms may also display the following *in vitro* dissolution rates when measured according to the European Pharmacopoeia paddle test at 50 rpm in 900 ml 0.1 N HCl pH 1.2 using UV detection at 270 nm:
10 to 30 % by weight of oxycodone or a salt thereof at 15 min,
40 to 60% by weight of oxycodone or a salt thereof at 2 h,
≥ 80% by weight of oxycodone or said salt thereof at 10 h,
10 to 30 % by weight of naloxone or a salt thereof at 15 min,
40 to 60% by weight of naloxone or a salt thereof at 2 h,
≥ 80% by weight of naloxone or said salt thereof at 10 h,

These release rates preferably apply if the dosage form comprises more than 20 mg of oxycodone, such as 40 mg of oxycodone and 20 mg of naloxone or 80 mg of oxycodone and. 40 mg of naloxone (or the equivalent amount of corresponding salts thereof).

In another embodiment the pharmaceutical dosage forms may display the following in vitro dissolution rates when measured according to the United States Pharmacopoeia Basket Method at pH 1.2 using UV detection at 270 nm:
15 to 30 % by weight of oxycodone or a salt thereof at 15 min,
45 to 70% by weight of oxycodone or a salt thereof at 2 h,
≥ 80% by weight of oxycodone or said salt thereof at 7 h,
≥ 90% by weight of oxycodone or said salt thereof at 12 h,
15 to 30 % by weight of naloxone or a salt thereof at 15 min,
45 to 70% by weight of naloxone or a salt thereof at 2 h,
≥ 80% by weight of naloxone or said salt thereof at 7 h,
≥ 90% by weight of naloxone or said salt thereof at 12 h.

The pharmaceutical dosage forms in accordance with the present invention which are to be used for treatment of Crohn's disease and which comprise oxycodone and naloxone or salts thereof may be formulated to allow for a therapeutic efficacy of preferably at least 12 hours and more preferably of at least 24 hours.

To this end, one can preferably rely on dosage formes which have been mentioned above. Thus, dosage forms comprising oxycodone and naloxone (or preferably their hydrochloride salts) and releasing these compounds in a sustained manner are preferred for this purpose. A weight ratio of oxycodone to naloxone (or their hydrochloride salts) of 2: 1 is particularly preferred. The aforementioned amounts such as 20 mg oxycodone and 10 mg naloxone can also be particularly preferred. These dosage forms may display the following *in vitro* dissolution rates when measured according to the European Pharmacopoeia paddle test at 50 rpm in 900 ml 0.1 N HCl pH 1.2 using UV detection at 270 nm:
10 - 30 % by weight of oxycodone or said salt thereof at 15 min,
30 - 50 % by weight of oxycodone or said salt thereof at 1 h,
40 - 65 % by weight of oxycodone or said salt thereof at 2 h,
60 - 85 % by weight of oxycodone or said salt thereof at 4 h,
70 - 95 % by weight of oxycodone or said salt thereof at 7 h,
≥ 80% by weight of oxycodone or said salt thereof at 10 h,
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

One of the morse preferred embodiments of the present invention relates to a pharmaceutical dosage form comprising oxycodone hydrochloride and naloxone hydrochloride in a weight ratio of oxycodone:naloxone of 2:1 and preferably at amounts of 20 mg oxycodone and 10 mg naloxone (or of the equivalents of corresponding saltes) with the dosage form displaying sustained release characteristics which are provided by a sustained release matrix that can preferably be made from a hydrophobic cellulose ether such as ethyl cellulose and/or a fatty alcohol and which provides the aforementioned following *in vitro* dissolution rates when measured according to the European Pharmacopoeia paddle test at 50 rpm in 900 ml 0.1 N HCl pH 1.2 using UV detection at 270 nm:
10 - 30 % by weight of oxycodone or said salt thereof at 15 min,
30 - 50 % by weight of oxycodone or said salt thereof at 1 h,
40 - 65 % by weight of oxycodone or said salt thereof at 2 h,
60 - 85 % by weight of oxycodone or said salt thereof at 4 h,
70 - 95 % by weight of oxycodone or said salt thereof at 7 h,
≥ 80% by weight of oxycodone or said salt thereof at 10 h,
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

The aforementioned dosage forms may be produced as described above by granulation or extrusion. As regards extrusion the aforementioned parameters can preferably be used.

Dosage forms comprising oxycodone and naloxone can also be characterized *inter alia* by their pharmacokinetic parameters.

Concentration gradients or blood plasma curves can be described by the parameters such as Cₘₐₓ, tₘₐₓ and AUC. These parameters are important in describing the pharmacokinetic properties of a specific drug formulation.

The Cₘₐₓ value indicates the maximum blood plasma concentration of the active agents, i.e. oxycodone and/or naloxone (or of their salts).

The tₘₐₓ value indicates the time point at which the Cₘₐₓ value is reached. In other words, tₘₐₓ, is the time point of the maximum observed plasma concentration.

The AUC (Area Under the Curve) value corresponds to the area of the concentration curve. The AUC value is proportional to the amount of active agents, i.e. oxycodone and naloxone absorbed into the blood circulation in total and is hence a measure for the bioavailability.

The AUCt value is the value for the area under the plasma concentration-time curve from the time of administration to the last measurable concentration. AUCt values are usually calculated using the linear trapezoidal method. Where possible, LambdaZ, which is the terminal phase rate constant, is estimated using those points determined to be in the terminal lock-linear phase. t1/2Z, which is the apparent terminal phase half-life, is commonly determined from the ratio of In2 to LambdaZ. The areas under the plasma concentration-time curve between the last measured points and infinity may be calculated from the ratio of the finial observed plasma concentration (Cₗₐₛₜ) to LambdaZ. This is then added to the AUCt to yield AUCinf, which is the area under the plasma concentration-time curve from the time of administration to infinity.

The term "bioavailability" is defined for purposes of the present invention as the extent to which active agents such as oxycodone and naloxone are absorbed from the unit dosage forms.

The term T_{1/2} is defined for purposes of the present invention as the amount of time necessary for one half of the absorbable dose of opioid agonist, preferably oxycodone, and opioid antagonist, preferably naloxone, to be transferred to plasma. This value may be calculated as a "true" value (which would take into account the effect of elimination processes), rather than an "apparent" absorption half-life.

Parameters describing the blood plasma curve can be obtained in clinical trials, first by once-off administration of the active agent such as oxycodone and naloxone to a number of test persons. The blood plasma values of the individual test persons are then averaged, e.g. a mean AUC, Cₘₐₓ and tₘₐₓ value is obtained. In the context, of the present invention, pharmacokinetic parameters such as AUC, Cₘₐₓ and tₘₐₓ refer to mean values. Further, in the context of the present invention, *in vivo* parameters such as values for AUC, Cₘₐₓ, tₘₐₓ, or analgesic efficacy refer to parameters or values obtained after administration at steady state or of a single dose to human patients and/or healthy human subjects.

If pharmacokinetic parameters such as mean tₘₐₓ, cₘₐₓ and AUC are measured for healthy human subjects, they are typically obtained by measuring the development of blood plasma values over time in a test population of approximately 16 to 24 healthy human subjects. Regulators bodies such as the European Agency for the Evaluation of Medicinal Products (EMEA) or the Food and Drug Administration (FDA) will usually accept data obtained from e.g. 20 or 24 test persons. However, initial trials involving fewer participants may also be acceptable.

The term "healthy" human subject in this context refers to a typical male or female of usually Caucasian origin with average values as regards height, weight and physiological parameters such as blood pressure etc. Healthy human subjects for the purposes of the present invention are selected according to inclusion and exclusion criteria which are based on and in accordance with recommendations of the International Conference for Harmonization of Clinical Trials (ICH). For the purposes of the present invention, healthy subjects may be identified according to the inclusion and exclusion criteria as outlaid in Example 7.

Thus, inclusion criteria comprise e.g. an age between ≥18 and ≤45 years; a BMI within the range 19-29 kg/m², and within the weight range 60-100 kg for males and 55 - 90 kg for females; that females must be non-nursing, non-pregnant, and provide a negative urine β-hCG pregnancy test within 24 hours before receiving the study medication; generally good health, evidenced by a lack of significantly abnormal findings on medical history, physical examination, clinical laboratory tests, vital signs, and ECG etc.

Exclusion criteria comprise e.g. exposure to any investigational drug or placebo within 3 months of the first dose of study medication, any significant illness within the 30 days before the first dose of study medication, any clinically significant abnormalities identified at prestudy screening for medical history, physical examination or laboratory analyses, use of any prescription medication (except HRT for postmenopausal females and contraceptive medication) in the 21 days, or over the counter medication including acid controllers, vitamins, herbal products and/or mineral supplements in the 7 days, before first dose of study medication, concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis), history of or concurrent medical condition, which in the opinion of the investigator would compromise the ability of the subject to safely complete the study, history of seizure disorders for which subjects required pharmacologic treatment, current history of smoking more than 5 cigarettes a day, subjects with evidence of active or past history of substance or alcohol abuse according to DSM-IV criteria, subjects who reported regular consumption of 2 or more alcoholic drinks per day or have blood alcohol levels of ≥0.5% at screening, donation of more than 500 mL of blood or blood products or other major blood loss in the 3 months before first dose of study medication, any positive results in the prestudy screen for ethanol, opiates, barbiturates, amphetamines, cocaine metabolites, methadone, propoxyphene, phencyclidine, benzodiazepines, and cannabinoids in the specimen of urine collected at screening, known sensitivity to oxycodone, naloxone, or related compounds etc.

If pharmacokinetic parameters such as mean tₘₐₓ, cₘₐₓ and AUC are obtained in patients, the patient group will comprise typically between 10 to 200 patients. A reasonable number of patients will e.g. be 10, 20, 30, 40, 50, 75, 100, 125 or 150 patients. Patients will be selected according to symptoms of the condition to be treated. For the purposes of the present invention, patients may be selected according to the inclusion and exclusion criteria of Example 7. Thus patients will be e.g.≥ 18 years, suffer from severe chronic pain of tumor and non-tumor origin, will show insufficient efficacy and/or tolerability with a WHO step II or III analgesic etc. A patient will not be considered for determination of pharmacokinetic parameters if there are indications of current alcohol or drug abuse, of current severe cardiovascular and respiratory diseases, of sever liver and renal insufficiency etc.

It is to be understood that values of pharmacokinetic parameters as indicated above and below have been deduced on the basis of the data which were obtained in Example 7, all of which relate to single dose studies in healthy human subjects. However, it is assured that comparable results will be obtained upon steady state administration in healthy human subject or single dose and steady state administration in human patients.

Pharmacokinetic parameter calculations may be performed with WinNonlin Enterprise Edition, Version 4.1.

The term "steady state" means that a plasma level for a given drug has been achieved and which is maintained with subsequent doses of the drug at a level which is at or above the minimum effective therapeutic level and is below the minimum toxic plasma level for oxycodone. For opioid analgesics such as oxycodone, the minimum effective therapeutic level will be partially determined by the amount of pain relief achieved in a given patient. It will be well understood by those skilled in the medical art that pain measurement is highly subjective and great individual variations may occur among patients. It is clear that after the administration of each dose the concentration passes through a maximum and then again drops to a minimum.

The steady state may be described as follows: At the time t = 0, the time the first dose is administered, the concentration C is also 0. The concentration then passes through a first maximum and then drops to a first minimum. Before the concentration drops to 0, another dose is administered, so that the second increase in concentration doesn't start at 0. Building on this first concentration minimum, the curve passes through a second maximum after the second dose has been administered, which is above the first maximum, and drops to a second minimum, which is above the first minimum. Thus, the blood plasma curve escalates due to the repeated doses and the associated step-by-step accumulation of active agent, until it levels off to a point where absorption and elimination are in balance. This state, at which absorption and elimination are in equilibrium and the concentration oscillates constantly between a defined minimum and a defined maximum, is called steady state.

The terns "maintenance therapy" and "chronic therapy" are defined for purposes of the present invention as the drug therapy administered to a patient after a patient is titrated with an opioid analgesic to a steady state as define above.

Preferably, or alternatively, the use of oxycodone/naloxone dosage forms according to the present invention provides a mean tₘₐₓ for oxycodone at about 1 to about 17 hours, at about 2 to about 15 hours, at about 3 to about 8 hours or at about 4 to about 5 hours after administration at steady state or of a single dose to human patients or healthy human subjects. In one preferred embodiment the dosage form provides a mean tₘₐₓ of 3 hours, 3.5 hours or 4.0 hours for oxycodone after administration at steady state or of a single dose to human healthy subjects or human patients. In a preferred embodiment such dosage forms comprise oxycodone and naloxone in a 2:1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxyeodone/naloxone dosage forms according to the present invention provides a mean tₘₐₓ for naloxone-3-glucuronide at about 0.25 to about 15 hours, at about 0.5 to about 12 hours, at about 1 to about 4 hours or at about 1 to about 3 hours after administration at steady state or of a single dose to human patients or healthy human subjects. In one preferred embodiment the dosage form provides a mean tₘₐₓ of 0.5 hour, 1 hour or 2.0 hours for naloxone-3-glucuronide after administration at steady state or of a single dose to human healthy subjects or human patients. In a preferred embodiment such dosage forms comprise oxycodone and naloxone in a 2: 1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone/naloxone dosage forms according to the present invention provides a mean A UCt value for oxycodone of about 100 ng·h/mL or about 200 ng·h/mL or about 300 ng·h/mL to about 600 ng·h/mL, more preferably about 400 ng·h/mL to about 550 ng·h/mL and most preferably from about 450 ng·h/mL to about 510 ng·h/mL. Preferably, these mean AUCt values for are achieved after administration at steady state or of a single dose to human healthy subjects or human patients. In a preferred embodiment such dosage forms comprise oxycodone and naloxone in a 2:1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone/naloxone dosage forms according to the present invention provides a mean AUCt value for naloxone-3-glucuronide of about 100 ng·h/mL or about 200 ng·h/mL or about 300 ng·h/mL to about 750 ng·h/mL, more preferably about 400 ng·h/mL to about 700 ng·h/mL and most preferably from about 500 ng·h/mL to about 600 ng·h/mL. Preferably, these mean AUCt values for naloxone-3-glucuronide are achieved after administration at steady state or of a single does to human healthy subjects or human patients. In a preferred embodiment such dosage forms comprise oxycodone and naloxone in a 2:1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone naloxone dosage forms according to the present invention provides a mean Cₘₐₓ value for oxycodone of about 5 ng/mL to about 50 ng/mL, more preferably of about 20 ng/mL to 40 ng/mL or most preferably of about 30 ng/mL to about 35 ng/mL. Preferably, these mean Cₘₐₓ values for oxycodone are achieved after administration at steady state or of a single does to human healthy subjects or human patients. In a preferred embodiment such dosage forms comprise oxycodone and naloxone in a 2:1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone/naloxone dosage forms according to the present invention provides a mean Cₘₐₓ value for oxycodone of about 0.125 ng/mL mg oxycodone to about 1.25 ng/mL mg oxycodone, more preferably of about 0.5 ng/mL mg oxycodone to 1 ng/mL mg oxycodone or most preferably of about 0.75 ng/mL mg oxycodone to about 0.875 ng/mL mg oxycodone. The above values relate to single dose administration or steady state administration in healthy human subjects or patients. In a preferred embodiment such dosage forms comprise oxycodone and naloxone in a 2:1 weight ratio. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone/naloxone dosage forms according to the present invention provides a mean Cₘₐₓ value for naloxone-3-glucuronide of about 10 pg/mL to about 100 pg/mL, more preferably of about 40 pg/mL to 90 pg/mL or most preferably of about 60 pg/mL of about 90 pg/mL. Preferably, these mean Cₘₐₓ values for oxycodone are achieved after administration of a single does to human healthy subjects or human patients. In a preferred embodiment such dosage forms comprise oxycodone and naloxone in a 2:1 weight ratio. These preparations are preferably administered up to a total amount of 40 mg oxycodone and 20 mg naloxone per day. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

Preferably, or alternatively, the use of oxycodone/naloxone dosage forms according to the present invention provides a mean Cₘₐₓ value for naloxone-3-glucuronide of about 2 pg/mL mg naloxone to about 4.5 pg/mL mg naloxone, more preferably of about 3 pg/mL mg naloxone to 4.5 pg/mL mg naloxone. The above values relate to single dose administration or steady state administration in healthy human subjects or patients. In a preferred embodiment such dosage forms comprise oxycodone and naloxone in a 2:1 weight ratio. Preferably, the dosage form comprises approximately 40 mg of oxycodone and 20 mg of naloxone and more preferably about 20 mg of oxycodone and 10 mg of naloxone. The dosage form preferably releases the agents in a sustained manner by the aforementioned release rates.

In one embodiment, the dosage form used according to the present invention contains oxycodone hydrochloride in an amount corresponding to 20 mg oxycodone, and naloxone hydrochloride in an amount corresponding to 10 mg naloxone.

For dosage forms containing the equivalent of 20 mg oxycodone and 10 mg naloxone it can be especially preferred that the sustained release is achieved by a sustained release matrix. Retardant materials can be selected from hydrophobic polymers such as cellulose ethers including ethyl cellulose and/or fatty alcohols including stearyl alcohol. In some specific embodiments, such dosage forms contain about 20-25% by weight fatty alcohol. Referred amounts for a cellulose ether in dosage forms according to this embodiment are about 7-9% by weight cellulose ether. The *in vitro* release rates mentioned above can be preferred.

In other embodiments, the dosage form contains oxycodone in an amount corresponding to 10 mg anhydrous oxycodone hydrochloride and naloxone in an amount corresponding to 5 mg naloxone hydrochloride. In this embodiment, it is also preferred that the retardant materials are selected from a cellulose ether and/or a fatty alcohol. The aforementioned weight ratios, retarding principle and *in vitro* release of the dosage form comprising 20 mg oxycodone and naloxone can be preferred.

In other preferred embodiments, the dosage forms used according to the present invention contain oxycodone in an amount corresponding to 40 mg oxycodone and naloxone salt in an amount corresponding to 20 mg naloxone. Again, the retardant materials are preferably selected from a cellulose ether and/or a fatty alcohol. The aforementioned weight ratios, retarding principle and *in vitro* release of the dosage form comprising 20 mg oxycodone and naloxone can be preferred.

The invention will now be illustrated with respect to examples which are, however, not to be construed as limiting.

### EMBODIMENT EXAMPLES

### Example 1: Production of tablets with different oxycodone/naloxone amounts in a non-swellable diffusion matrix by spray granulation

The following amounts of the listed components were used for the production of oxycodone/naloxone tablets according to the invention.

**Table 1**

| Preparation (designation) | OXN_1 | OXN_2 | OXN_3 |
|---|---|---|---|
| Oxycodone HCl | 20.0 mg | 20.0 mg | 20.0 mg |
| Naloxone HCl | - | 5.0 mg | 10.0 mg |
| Lactose Flow Lac 100 | 59.25 mg | 54.25 mg | 49.25 mg |
| Povidone 30 | 5.0 mg | 5.0 mg | 5.0 mg. |
| Surelease® | 10.0 mg solid material | 10.0 mg solid material | 10.0 mg solid material |
| Stearyl alcohol | 25.0 mg | 25.0 mg | 25.0 mg |
| Talcum | 2.5 mg | 2.5 mg | 2.5 mg |
| Mg-Stearate | 1.25 mg | 1.25 mg | 1.25 mg |

The Surelease® E-7-7050 polymer mixture used had the following composition.

**Table 2**

| Stirelease ® |
|---|
| Ethylcellulose 20 cps |
| Dibutylsebacate |
| Ammoniumhydroxide |
| Oleic acid |
| Siliciumdioxide |
| Water |

For the production of tablets oxycodone HCl, naloxone HCl, Povidone 30 and Lactose Flow Lac 100 were mixed in a tumbling mixer (Bohle) and subsequently spray-granulated with Surelease® E-7-7050 in a fluidized bath granulating device (GPCG3). The material was sieved over a Comill 1.4 mm sieve. An additional granulation step was carried out with melted fatty alcohol in a high-shear mixer (Collette). All tablet cores produced by this approach had a weight of 123 mg, based on dry substance.

### Example 2: Production of tablets with oxycodone and naloxone in a non-swellable diffusion matrix by extrusion

The following amounts of the listed components were used for the production of the oxycodone/naloxone tablets according to the invention.

**Table 3**

| Preparation (designation) | OXN_4 |
|---|---|
| Oxycodone HCl | 20 mg |
| naloxone HCl | 10 mg |
| Kollidon 30 | 6 mg |
| Lactose Flow Lac 100 | 49.25 mg |
| Ethylcellulose 45 cpi | 10 mg |
| Stearyl alcohol | 24 mg |
| Talcum | 2.5 mg |
| Mg-Stearate | 1,25 mg |

The listed amounts of oxycodone HCl, naloxone HCl, ethylcellulose 45 cpi, Povidone 30, stearyl alcohol and Lactose Flow Lac 100 were mixed in a tumbling mixer (Bohle). This mixture was subsequently extruded with a counter-rotating twin screw extruder of the type Micro 18 GGL (Leistritz AG, Nürnberg, Germany). The temperature of heating zone 1 was 25°C, of heating zone 2, 50°C, of heating zones 3 to 5,60°C, of heating zones 6 to 8, 55°C, of heating zone 9, 60°C and of heating zone 10,65°C. The screw rotating speed was 150 revolutions per minute (rpm), the resulting melt temperature was 87°C, the feed rate was 1.5 kg/h and the diameter of the nozzle opening was 3 mm. The extruded material was sieved with a Frewitt 0.68 x 1.00 mm sieve. The grinded extrudate was then mixed with talcum and magnesium stearate that had been added over a 1 mm hand sieve and was subsequently pressed into tablets.

In comparison to the oxycodone/naloxone tablets which also have the Surelease®-based non-swellable diffusion matrix produced by spray granulation (see Example 1), extruded preparations comprise less components.

### Example 3: Release profile of the oxycodone/naloxone tablets from Example 1

The release of the active compounds was measured over a time period of 12 hours, applying the Basket Method according to USP at pH 1.2 using HPLC. Tablets OXN_1, OXN_2 and OXN_3 were tested.

The release rates of different oxycodone amounts, independent of the naloxone amount, remain equal (invariant). Correspondingly, invariant release profiles are observed for naloxone at different oxycodone amounts.

**Table 4**

| Time (min) | OXN_1 | OXN_2 | OXN_2 | OXN_3 | OXN_3 |
|---|---|---|---|---|---|
| | Oxyc. | Oxyc. | Nal. | Oxyc. | Nal. |
| 0 | 0 | 0 | 0 | 0 | 0 |
| 15 | 26.1 | 24.9 | 23.5 | 22.8 | 24.1 |
| 120 | 62.1 | 63 | 61 | 57.5 | 60.2 |
| 420 | 91.7 | 94.5 | 91.9 | 89.4 | 93.5 |
| 720 | 98.1 | 99.6 | 96.6 | 95.7 | 100.6 |

The release values refer to oxycodone or naloxone (line 2) and are given as percentages. Oxyc. and Nal. stand for oxycodone and naloxone and indicate the compound measured.

### Example 4: Release profile of oxycodone/naloxone tablets from Example 2 at different pH-values

The release of active compounds from the tablets was measured over a time period of 12 hours at pH 1.2 or for 1 hour at 1.2 and subsequently for 11 hours at pH 6.5. Release rates were determined by the basket method according to USP using HPLC.

The following release rates were measured for 12 hours at pH 1.2:

**Table 5**

| Time (min) | OXN_4 | OXN_4 |
|---|---|---|
| | Oxyc. | Nal. |
| 0 | 0 | 0 |
| 15 | 24.1 | 24.0 |
| 120 | 62.9 | 63.5 |
| 420 | 92.9 | 93.9 |
| 720 | 96.9 | 98.1 |

The following release rates were measured for 1 hour at pH 1.2 and 11 hours at pH 6.5:

**Table 6**

| Time (min) | OXN_4 | OXN_4. |
|---|---|---|
| | Oxyc. | Nal. |
| 0 | 0 | 0 |
| 60 | 48.1 | 49.2 |
| 120 | 65.0 | 64.7 |
| 240 | 83.3 | 81.8 |
| 420 | 94.1 | 92.3 |

The release rates refer to oxycodone and naloxone (line 2) and are given as percentages. Oxyc. and Nal. stand for oxycodone and naloxone and indicate the compound measured.

### Example 5: Production of tablets with different oxycodone/naloxone amounts in a non-swellable diffusion matrix by extrusion

The following amounts of the listed components were used for the production of oxycodone/naloxone tablets according to the invention.

**Table 7**

| Preparation (designation) | OXN_5 | OXN_6 | OXN_7 | OXN_8 |
|---|---|---|---|---|
| Oxycodone HCl | 20 mg | 20 mg | 20 mg | 20 mg |
| Naloxone HCl | 1 mg | 1 mg | 1 mg | 10 mg |
| Lactose Flow | 58.25 mg | 58.25 mg | 58.25 mg | 49.25 mg |
| Lac 100 | | | | |
| Kollidon® 30 | 6 mg | 6 mg | 6 mg | 6 mg |
| Ethylcellulose | 10 mg | 10 mg, | 10 mg | 10 mg |
| Stearly alcohol | 24 mg | 24 mg | 24 mg | 24 mg |
| Talcum. | 1.25mg | 1.25 mg | 1.25 mg | 1.25 mg |
| Mg-Stearate | 2.5 mg | 2.5 mg | 2.5 mg | 2.5 mg |

Extrusion was performed as described above (Example 2) with the following parameters:

| | | |
|---|---|---|
| OXN_5: | temperature: | 55-63 °C |
| | rpm (screw): | 150 rpm |
| | feeding rate: | 1.5 kg/h |
| | | |
| OXN_6: | temperature: | 55-63 °C |
| | rpm (screw): | 155 rpm |
| | feeding rate: | 1.5 kg/h |
| | | |
| OXN_7: | temperature: | 55-63 °C |
| | rpm (screw): | 155 rpm |
| | feeding rate: | 1.5 kg/h |
| | | |
| OXN_18: | temperature: | 55-63 °C |
| | rpm (screw): | 160 rpm |
| | feeding rate: | 1.75 kg/h |

Tablet production was performed with a common tabletting device with the following parameters:

| | | |
|---|---|---|
| OXN_5: | rpm: | 40 rpm |
| | Pressure power: | 9 kN |
| | | |
| OXN_6: | rpm: | 42 rpm |
| | Pressure power: | 8.9 kN |
| | | |
| OXN_7: | rpm: | 36 rpm |
| | Pressure power: | 9 kN |
| | | |
| OXN_8: | rpm: | 36 rpm |
| | Pressure power: | 7.5 kN |

The release of the active compounds was measured over a time period of 12 hours, applying the Basket Method according to USP at pH 1.2 using HPLC. Tablets OXN_5, OXN_6, OXN_7 and OXN_8 were tested.

**Table 8**

| Time (min) | OXN_5 | | OXN_6 | | OXN_7 | | OXN_8 | |
|---|---|---|---|---|---|---|---|---|
| | Oxyc. | Nal. | Oxyc. | Nal. | Oxyc. | Nal. | Oxyc. | Nal. |
| 0 | 0 | 0 | 0 | 0 | 0 | 0 | 0 | 0 |
| 15 | 21.2 | 25.8 | 21.7 | 21.1 | 19.7 | 19.3 | 23.3 | 24.3 |
| 120 | 56.6 | 53.8 | 58.8 | 57.3 | 57.7 | 56.2 | 64.5 | 66.9 |
| 420 | 87.2 | 84.5 | 94.2 | 92.6 | 93.7 | 91.5 | 92.7 | 96.3 |
| 720 | 99.7 | 96.8 | 100.1 | 98 | 100.6 | 97.5 | 93.6 | 97.4 |

The release values refer to oxycodone or naloxone (line 2) and are given as percentages. Oxyc. and Nal. stand for oxycodone and naloxone and indicate the active compound which has been measured.

### Example 6: Production of tablets with oxycodone/naloxene in a non-swellable diffusion matrix by extrusion

In the following example it is set out that using formulations according to the present invention, preparations comprising oxycodone and naloxone with particular release behaviours may be obtained.

The following amounts of the listed components were used for the production of oxycodone/naloxone tablets according to the invention.

**Table 9**

| Preparation (designation) | OXN_9 | OXN_10 | OXN_1 | OXN_12 | OXN_13 | OXN_14 |
|---|---|---|---|---|---|---|
| Oxycodone HCl | 20 mg, | 20 mg | 20 mg | 20 mg | 20 mg | 20 mg |
| naloxone HCl | 1 mg | 1 mg | 10 mg | 10 mg | 10 mg | 10 mg |
| Lactose Flow | 56.25 mg | 56.25 mg | 54.25 mg | 65.25 mg | 60.25 mg | 55.25 |
| Lac 100 | | | | | | |
| Kollidon® 30 | 7 mg | 6 mg | 6 mg | 7.25 mg | 7.25 mg | 7.25 mg |
| Ethylcellulose | 11 mg | 12 mg | 10 my | 12 mg | 12 mg | 12 mg |
| Stearyl alcohol | 24 mg | 24 mg | 24 mg | 28.75 mg | 28.75 mg | 28.75 mg |
| Talcum | 1.25 mg | 1.25 mg | 1.25 mg | 1.25 mg | 1.25 mg | 1.25 mg |
| Mg-Stearate | 2.5 mg | 2.5 mg | 2.5 mg | 2.5 mg | 2.5 mg | 2.5 mg |

Extrusion was performed as described above with the following parameters:

| | | |
|---|---|---|
| OXN_9: | temperature: | 55-63 °C |
| | rpm (screw): | 150 rpm |
| | feeding rate: | 1.5 kg/h |
| | | |
| OXN_10: | temperature: | 55-63 °C |
| | rpm (screw): | 150 rpm |
| | feeding rate: | 1.5 kg/h |
| | | |
| OXN_11: | temperature: | 55-63 °C |
| | rpm (screw): | 160 rpm |
| | feeding rate: | 1.75 kg/h |
| | | |
| OXN_12: | temperature: | 55-63 °C |
| | rpm (screw): | 160 rpm |
| | feeding rate: | 1.75 kg/h |
| | | |
| OXN_13: | temperature: | 55-63 °C |
| | rpm (screw): | 150 rpm |
| | feeding rate: | 1.5 kg/h |
| | | |
| OXN_14: | temperature: | 55-63 °C |
| | rpm (screw): | 150 rpm |
| | feeding rate: | 1.5 kg/h |

Tablet production was performed with a common tabletting device with the following parameters:

| | | |
|---|---|---|
| OXN_9: | rpm: | 39 rpm |
| | Pressure power: | 11 kN |
| | | |
| OXN_10: | rpm: | 39rpm |
| | Pressure power: | 10.5 kN |
| | | |
| OXN_11: | rpm: | 36 rpm |
| | Pressure power: | 9.5 kN |
| | | |
| OXN_12: | rpm: | 36 rpm |
| | Pressure power: | 7.8 kN |
| | | |
| OXN_13: | rpm: | 39rpm |
| | Pressure power: | 9 kN |
| | | |
| OXN_14: | rpm: | 39 rpm |
| | Pressure power: | 7.5 kN |

The release of the active compounds was measured over a time period of 12 hours, applying the Basket Method according to USP at pH 1.2 using HPLC. Tablets OXN_9, OXN_10, OXN_11, OXN_12, OXN_13 and OXN_14 were tested.

**Table 10**

| Time (min) | OXN_9 | | OXN_10 | | OXN_11 | | OXN_12 | | OXN_13 | | OXN_14 | |
|---|---|---|---|---|---|---|---|---|---|---|---|---|
| | Oxyc. | Nal | Oxyc. | Nal | Oxyc. | Nal | Oxyc. | Nal | Oxyc. | Nal | Oxyc. | Nal |
| 0 | 0 | 0 | 0 | 0 | 0 | 0 | 0 | 0 | 0 | 0 | 0 | 0 |
| 15 | 16.6 | 16.2 | 17.4 | 17.2 | 26.1 | 26.8 | 21.8 | 21.9 | 18.5 | 18.2 | 18.4 | 18.2 |
| 120 | 47.6 | 46.9 | 49.6 | 49.7 | 71.1 | 73.0 | 61.2 | 61.8 | 52.8 | 52.8 | 53.3 | 53.3 |
| 420 | 82.7 | 84.5 | 84.6 | 85.7 | 94.3 | 96.6 | 93.2 | 94.7 | 86.3 | 86.3 | 87.2 | 88.2 |
| 720 | 95 | 97 | 95.2 | 95.8 | 94.9 | 97.9 | 96.4 | 97.9 | 94.8 | 94.8 | 95.7 | 96.5 |

The release values refer to oxycodone or naloxone (line 2) and are given as percentages. Oxyc. and Nal. stand for oxycodone and naloxone and indicate the active compound which has been measured.

### Example 7: Pharmacokinetic and bioavailability characteristics of different strengths of a fixed combination of oxycodone and naloxone and a combination of Oxygesic® plus Naloxone CR

### 1. Objective

The objectives of this study were to (i) evaluate the pharmacokinetic and bioavailability parameters of oxycodone and naloxone and their main metabolites when administered as a controlled-release fixed combination tablet formulation; (ii) assess the interchangeability between the 3 different strengths of the fixed combination, OXN 10/5, OXN 20/10 and OXN 40/20; and (iii) compare the pharmacokinetics and bioavailability of the fixed combination formulation with marketed Oxygesic® given together with Naloxone CR tablets;

### 2. Test population

A total of 28 healthy adult, male and female subjects were randomized to receive the study drugs with the aim that 24 subjects would complete the study and provide valid pharmacokinetic data.

### Inclusion Criteria

Subjects who were included in the study were those who met all of the following criteria:
- Males or females of any ethnic group;
- Aged between ≥18 and ≤45 years;
- BMI within the range 19 - 29 kg/m², and within the weight range 60 - 100 kg for males and 55 - 90 kg for females;
- Females must be non-nursing, non-pregnant, and provide a negative urine β-hCG pregnancy test within 24 hours before receiving the study medication. Female subjects of childbearing potential must be using a reliable form of contraception (e.g. intrauterine device, oral contraceptive, barrier method). Female subjects who were postmenopausal must have been postmenopausal for ≥1 year and, in the absence of HRT, have elevated serum FSH;
- Generally good health, evidenced by a lack of significantly abnormal findings on medical history, physical examination, clinical laboratory tests, vital signs, and ECG. Vital signs (after 3 minutes resting in a supine position) must be within the following ranges: oral body temperature between 35.0 - 37.5 °C; systolic blood pressure, 90 - 140 mmHg; diastolic blood pressure, 50 - 90 mmHg; and pulse rate, 40 - 100 bpm. Blood pressure and pulse were taken again after 3 minutes in a standing position. After 3 minutes standing from a supine position, there should be no more than a 20 mmHg drop in systolic blood pressure, 10 mmHg drop in diastolic blood pressure, and no greater than 20 bpm increase in pulse rate; Written informed consent obtained; Willing to eat all the food supplied during the study.

### Exclusion Criteria

Subjects who were excluded from the study were those who met any of the following criteria:
- Exposure to any investigational drug or placebo within 3 months of their first dose of study medication;
- Any significant illness within the 30 days before their first dose of study medication;
- Any clinically significant abnormalities identified at prestudy screening for medical history, physical examination or laboratory analyses;
- Use of any prescription medication (except HRT for postmenopausal females and contraceptive medication) in the 21 days, or over the counter medication including acid controllers, vitamins, herbal products and/or mineral supplements in the 7 days, before their first dose of study medication;
- Concurrent medical condition known to interfere with gastrointestinal drug absorption (e.g. delayed gastric emptying, mal absorption syndromes), distribution (e.g. obesity), metabolism or excretion (e.g. hepatitis, glomerulonephritis);
- History of, or concurrent medical condition, which in the opinion of the Investigator would compromise the ability of the subject to safely complete the study;
- History of seizure disorders for which subjects required pharmacologic treatment;
- Current history of smoking more than 5 cigarettes a day;
- Subjects with evidence of active or past history of substance or alcohol abuse, according to DSM-IV criteria3, or subjects who. In the investigator's opinion, have demonstrated addictive or substance abuse behaviors;
- Subjects who reported regular consumption of 2 or more alcoholic drinks per day or have blood alcohol levels of ≥0.5% at screening;
- Donation of more than 500 mL of blood or blood products or other major blood loss in the 3 months before their first dose of study medication;
- At risk of transmitting infection via blood samples such as producing a positive HIV test at screening or having participated in a high risk activity for contracting HIV; producing a positive Hepatitis B surface antigen test at screening; producing a positive Hepatitis C antibody test at screening;
- Any positive results in the prestudy screen for ethanol, opiates, barbiturates, amphetamines, cocaine metabolites, methadone, propoxyphene, phencyclidine, benzodiazepines, and cannabinoids in the specimen of urine collected at screening;
- Known sensitivity to oxycodone, naloxone, or related compounds;
- Contraindications and precautions as detailed in the datasheet for Oxygesic@;
- Refusal to allow their primary care physician (if applicable) to be informed;
- The Investigator believed the subject to be unsuitable for a reason not specifically stated in the exclusion criteria.

The demographic data are shown in Table 11.

**Table 11: Subject Demographics and Other Baseline Characteristics: Safety Population**

| | Male | Female | Overall |
|---|---|---|---|
| | (N = 22) | (N = 6) | (N = 28) |
| CHARACTERISTICS | | | |
| Race, n (%) | | | |
| Caucasian | 22 (100%) | 6 (100%) | 28 (100%) |
| Age (y) | | | |
| Mean ± SD | 32.6 ± 5.28 | 31.0 ± 6.32 | 32.3 ± 5.44 |
| Range (min, max) | 25,41 | 24,42 | 24,42 |
| Height (cm) | | | |
| Mean ± SD | 179.1+4.84 | 168.0 ± 8.72 | 176.7 ± 7.33 |
| Range (min, max) | 165,187 | 159,181 | 159,187 |
| Weight (kg) | | | |
| Mean ± SD | 77.8 ± 9.04 | 67.0 ± 3.03 | 75.5 ± 9.25 |
| Range (min, max) | 62,97 | 63,71 | 62,97 |
| Body Mass Index (kg/m²) | | | |
| Mean ± SD | 24.2 ± 2.56 | 23.9 ± 2.50 | 24.2 ± 2.50 |
| Range (min, max) | 20,29 | 20,27 | 20,29 |

### 3. Study Design, Test Treatment Dose and Mode of Administration

### Preparation of tested products

A melt extrusion oxycodone/naloxone controlled-release tablet formulation with an oxycodone:naloxone ratio of 2:1 was produced. There are three dose strengths available, namely OXN 10/5, OXN 20/10, and OXN 40/20, where the first number is the mg amount of oxycodone hydrochloride and the second number is the mg amount of naloxone hydrochloride (see Table 12). OXN 20/10 and OXN 40/20 are from the same granulate, while OXN 10/5 has a slightly different formula in regard to the ratio of active ingredients to excipients.

Oxycodone/naloxone tablets (OXN Tablets) according to this example contain a fixed combination of oxycodone and naloxone in the ratio of 2:1. Tablets formulations are summarized below (see Table 12).

The 20/10 mg and 40/20 mg tablets are manufactured from the same granulation with these two tablet strengths being compositionally proportional. Oxycodone/Naloxone prolonged release tablets (OXN) tablets according to this example are controlled release tablets using a matrix of stearyl alcohol and ethylcellulose as the retardant. The tablets contain the combination of oxycodone hydrochloride and naloxone hydrochloride in the strengths 10/5 mg, 20/10 mg and 40/20 mg (both as the hydrochloride). The complete statement of the components and quantitative composition of Oxycodone/Naloxone prolonged release tablets is given below in Table 12.

**Table 12: Oxycodone/Naloxone prolonged release tablets.**

| **Component** | **Quantity (mg/tablet)** | | | **Function** | **Reference to Standard** |
|---|---|---|---|---|---|
| | OXN 10/5 | OXN 20/10 | OXN 40/20 | | |
| Oxycodone hydrochloride 1) | 10.50 | 21.00 | 42.00 | Active | USP*/ H.S.E. |
| *corresponding to* | | | | | |
| *Oxycodone hydrochloride anhydrous* | *10.00* | *20.00* | *40.00* | | |
| *Oxycodone base* | *9.00* | *18.00* | *36.00* | | |
| Naloxone hydrochloride Dihydrate | 5.45 | 10.90 | 21.80 | Active | Ph. Eur.* |
| *corresponding to* | | | | | |
| *Naloxone hydrochloride anhydrous Naloxone base* | *5.00* | *10.00* | *20.00* | | |
| | *4.50* | *9.00* | *18.00* | | |
| Povidone K30 | 5.00 | 7.25 | 14.50 | Binder | Ph. Eur.* |
| Ethylcellulose N 45 | 10.00 | 12.00 | 24.00 | Retardant | Ph. Eur.* |
| Stearyl alcohol | 25.00 | 29.50 | 59.00 | Retardant | Ph. Eur.* |
| Lactose monohydrate | 64.25 | 54.50 | 109.00 | Diluent | Ph. Eur.* |
| Purified talc | 2.50 | 2.50 | 5.00 | Glidant | Ph. Eur.* |
| Magnesium stearate | 1.25 | 1.25 | 2.50 | Lubricant | Ph. Eur.* |
| **Total core** | **123.95** | **138.90** | **277.80** | | |
| **Film Coat°** | | | | | |
| Opadry II HP white - 85F18422 | 3.72 | | | Coating | supplier specificati on |
| Opadry II HP pink - 85F24151 | | 4.17 | | Coating | supplier specificati on |
| Opadry II HP yellow 85F32109 | | | 8.33 | Coating | supplier specificati on |
| Purified talc | 0.12 | 0.14 | 0.28 | Gloss | Ph. Eur.* |
| **Total film tablet** | **127,79** | **143.21** | **286.41** | | * current Edition |

| | | | | | |
|---|---|---|---|---|---|
| ¹⁾ calculated based on expected moisture content ° qualitative composition : see Table 12 | | | | | |

**Table 13: Qualitative composition of the film coat.**

| O**padry II HP** | **white 85F18422** | **pink 85F24151** | **yellow 85F32109** | **Reference to Standard** |
|---|---|---|---|---|
| Polyvinylalcohol part. hydrolized | + | + | + | Ph. Eur. * |
| Titanium dioxide (E 171) | + | + | + | Ph. Eur. * |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellow (E 172) | | | + | NF* /EC Directive |
| | | | | * current Edition |

### Study Design

The study was an open-label, single dose, 4-treatment, 4-period, randomized across over study and healthy subjects. The treatments were given orally in the fasted state as follows:
- Treatment A: 4 x tablets of Oxn 10/5
- Treatment B: 2 x tablets of Oxn 20/10
- Treatment C: 1 x tablets of Oxn 40/20

The reference treatment was an Oxygesic® 20 mg tablet. Naloxone was used in the form of Naloxone 10 mg CR spray granulation tablet. Reference treatment was thus

- Treatment D: 2 tablets of Oxygesic® 20 mg and two tablets of Naloxone CR 10 mg.

Duration of treatment included 21 days screening period and four study periods each with a single dose of study drug followed by a seven day wash-out period. There were post study medical 7 to 10 days after dosing of study period 4 and there were 7 to 10 days after discontinuation from the study. The total duration was 49 to 52 days.

The treatment schedule was a single dose of study drug in each of the four study periods. Each dose of study drug was separated by a 7 day wash-out period.

The enrolled population was defined as the subject population that provided the written informed consent to anticipate in the study. The full analysis population for pharmacokinetics was defined as those subjects, who had at least one valid pharmacokinetic parameter calculated on at least one treatment.

### 4. Pharmacokinetic Assessments

### Drug Concentration Measurements

Blood samples for determining oxycodone, noroxycodone, oxymorphone, noroxymorphone, naloxone, 6β-naloxol and naloxone-3-glucuronide concentrations were obtained for each subject during each of the 4 study periods immediately before dosing; and at 0.5, 1, 1.5, 2, 2.5, 3, 3.5, 4, 5, 6, 8, 10, 12, 16, 24, 28, 32, 36, 48, 72 and 96 hours (22 blood samples per study period) after dosing. Blood was also drawn where possible at the first report of a serious or severe unexpected adverse event and at its resolution.

At each time of plasma determination, 6 mL venous blood was drawn from a forearm vein into a tube containing K2 EDTA anticoagulant. All samples were processed according to common sample handling procedures.

### Pharmacokinetic Parameters

The following pharmacokinetic parameters were calculated from the plasma concentrations of oxycodone, noroxycodone, oxymorphone, noroxymorphone, naloxone, 6β-naloxol and naloxone-3-glucuronide:
- Area under the plasma concentration time curve calculated to the last measurable concentration (AUCt);
- Area under the plasma concentration-time curve, from the time of administration to infinity (AUCINF);
- Maximum observed plasma concentration (Cₘₐₓ);
- Time point of maximum observed plasma concentration (tₘₐₓ);
- Terminal phase rate constant (LambdaZ);
- Apparent terminal phase half life (t½Z).
   For oxycodone, noroxycodone, oxymorphone, noroxymorphone, and naloxone-3-glucuronide, AUC values were given in ng·h/mL, and Cₘₐₓ values in ng/mL. For naloxone and 6β-naloxol, AUC values, due to the low concentrations, were given in pg·h/mL and Cₘₐₓ values in pg/mL.
   AUCt, AUCINF and Cₘₐₓ were regarded as the primary parameters.
   AUCt were calculated using the linear trapezoidal method. Where possible, LambdaZ was estimated using those points determined to be in the terminal log-linear phase. t½Z was determined from the ratio of ln 2 to LambdaZ. The areas under the plasma concentration-time curve between the last measured point and infinity were calculated from the ratio of the final observed plasma concentration (Cₗₐₛₜ) to LambdaZ. This was then added to the AUCt to yield AUCINF.
   All pharmacokinetic calculations were performed with WinNonlin Enterprise Edition, Version 4.1.

### Statistical Methods

Cₘₐₓ and AUCINF of oxycodone were important in order to assess the equivalence of the 4 treatments. AUCt was calculated using the linear trapezoidal Method. Where possible, LambdaZ was estimated using those points determined to be in the terminal log-linear phase. t½Z were determined from the ratio of ln 2 to LambdaZ. The areas under the plasma concentration-time curve between the last measured point and infinity were calculated from the ratio of the final observed plasma concentration (Cₗₐₛₜ) to LambdaZ. This was added to the AUCt to yield the area under the plasma concentration-time curve between the time of administration and infinity (AUCINF).

The dose adjusted relative systemic availabilities (Frelt, and FrelINF) and the Cₘₐₓ ratio were obtained from the ratio of AUCt, AUCINF and Cₘₐₓ values, respectively, for differences defined in the following comparisons of interest:
fixed combination A vs. open combination D
fixed combination B vs. open combination D
fixed combination C vs. open combination D
fixed combination A vs. fixed combination B
fixed combination A vs. fixed combination C
fixed combination B vs. fixed combination C

The full analysis population for pharmacokinetics were used for these analyses.

The metabolite: parent drug AUCt and AUCINF ratios were estimated for each treatment, where possible.

### 5. Clinical Pharmacology Results

Mean observed plasma concentration - time curves for oxycodone, naloxone-3-glucuronide, naloxone, noroxycodone, oxymorphone, noroxymorphone and 6-β-naloxol are presented in Figures 1 to 7.

Pharmacokinetic parameters for oxycodone, naloxone-3-glucuronide and naloxone are presented in Tables 14 to 19 respectively.

**Table 14: Summary of Pharmacokinetic Parameters for Oxycodone by Treatment: Full Analysis Population for Pharmacokinetics**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (ng.h/mL)** | | | | |
| N | 24 | 23 | 23 | 23 |
| Arithmetic Mean | 473.49 | 491.22 | 489.89 | 502.28 |
| (SD) | (72.160) | (82.181) | (91.040) | (84.128) |
| Geometric Mean | 468.29 | 484.58 | 481.08 | 495.72 |
| **AUCINF (ng.h/mL)** | | | | |
| N | 24 | 22 | 22 | 22 |
| Arithmetic Mean | 475.06 | 497.17 | 491.22 | 509.11 |
| (SD) | (72.182) | (81.687) | (93.458) | (82.963) |
| Geometric Mean | 469.87 | 490.65 | 483.04 | 502.80 |
| **Cmax (ng/mL)** | | | | |
| N | 24 | 23 | 23 | 23 |
| Arithmetic Mean | 34.91 | 35.73 | 34.46 | 40.45 |
| (SD) | (4.361) | (4.931) | (5.025) | (4.706) |
| Geometric Mean | 34.66 | 35.41 | 34.12 | 40.19 |
| **tmax (h)** | | | | |
| N | 24 | 23 | 23 | 23 |
| Median | 3.5 | 4.0 | 3.0 | 2.5 |
| (Min,Max) | (1.0, 6.0) | (2.0, 8.0) | (1.0, 6.0) | (0.5, 8.0) |
| **t1/2Z** | | | | |
| N | 24 | 22 | 22 | 22 |
| Arithmetic Mean | 4.69 | 4.87 | 4.83 | 5.01 |
| (SD) | (0.775) | (0.995) | (0.975) | (0.802) |

**Table 15. Oxycodone Summary of Ratios for AUCt, AUCINF, Cₘₐₓ and Differences for tₘₐₓ and Half-Life - Full Analysis Population for Pharmacokinetics.**

| **Pharmacokinetic metric** | **4 x OXN 10/5 / 2 x Oxygesic 20 + 2 x Naloxone 10** | **2 x OXN 20/10 / 2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5/1 x OXN 40/20** | **2 x OXN 20/10 / 1 x OXN 40/20** |
|---|---|---|---|---|---|---|
| **AUCt (ng.h/mL)** | | | | | | |
| Ratio (%) | 94.9 | 98.2 | 98.0 | 96.7 | 96.8 | 100.2 |
| 90%CI | 91.5, 98.5 | 94.5, 102,0 | 94.4, 101.7 | 93.1, 100.4 | 93.3, 100.5 | 96.5, 104.0 |
| **AUCINF-(ng.h/m** | | | | | | |
| L) | | | | | | |
| Ratio (%) | 94.5 | 98.2 | 97.8 | 96.2 | 96.5 | 100.4 |
| 90%CI | 90.9, 98.1 | 94.5, 102.1 | 94.1, 101.7 | 92.6, 99.9 | 92.9, 100.3 | 96.5, 104.3 |
| **Cmax (ng/mL)** | | | | | | |
| Ratio (%) | 86.2 | 88.4 | 85.8 | 97.5 | 100.5 | 103.1 |
| 90%CI | 82.2, 90.4 | 84.2, 92.8 | 81.8, 90.0 | 92.9, 102.3 | 95.8, 105.4 | 98.2, 108.1 |
| **tmax (h)** | | | | | | |
| Difference | 0.49 | 1.11 | 0.14 | -0.63 | 0.35 | 0.97 |
| 90%CI | -0.19, 1.16 | 0.42, 1.80 | -0.54, 0.82 | -1.31, 0.05 | -0.33, 1.02 | 0.29, 1.66 |
| **t1/2Z (h)** | | | | | | |
| Difference | -0.27 | -0.11 | -0.11 | -0.16 | -0.16 | 0.00 |
| 90%Cl | -0.60, 0.05 | -0.44, 0,23 | -0.44,0.22 | -0.49, 0.16 | -0.49, 0.16 | -0.33, 0.33 |

**Table 16. Summary of Pharmacokinetic Parameters for Naloxone-3-glucuronide by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt (pg.h/mL)** | | | | |
| N | 24 | 23 | 23 | 23 |
| Arithmetic Mean | 539.93 | 522.45 | 520.10 | 523.37 |
| (SD) | (142.241) | (128.569) | (133.175) | (119.752) |
| Geometric Mean | 520.14 | 506.63 | 502.26 | 509.38 |
| **AUCINF(pg.h/mL)** | | | | |
| N | 22 | 21 | 22 | 22 |
| Arithmetic Mean | 562.53 | 520.97 | 527.94 | 537.25 |
| (SD) | (130.732) | (133.172) | (135.424) | 110.829 |
| Geometric Mean | 546.73 | 504.34 | 509.62 | 525.91 |
| **Cmax (pg/mL)** | | | | |
| N | 24 | 23 | 23 | 23 |
| Arithmetic Mean | 62.01 | 63.62 | 61.95 | 63.55 |
| (SD) | (15.961) | (19.511) | (18.369) | (16.748) |
| Geometric Mean | 59.93 | 60.70 | 59.34 | 61.55 |
| **tmax (h)** | | | | |
| N | 24 | 23 | 23 | 23 |
| Median | 1.0 | 0.5 | 1.0 | 1.0 |
| (Min,Max) | (0.5,3.0) | (0.5,6.0) | (0.5,3.0) | (0.5,6.0) |
| **t1/2Z** | | | | |
| N | 22 | 21 | 22 | 22 |
| Arithmetic Mean | 8.48 | 7.93 | 7.81 | 7.66 |
| (SD) | (3.066) | (2.402) | (2.742) | (1,717) |

**Table 17. Naloxone-3-Glucuronide Summary of Ratios for AUCt, AUCINF, Cₘₐₓ and Differences for Tₘₐₓ and Half-Life- Full Analysis Population for Pharmacokinetics.**

| **Pharmacokinetic metric** | **4 x OXN 10/5/ 2x Oxygesic 20 + 2 x Naloxone 10** | **2 x OXN 20/10 / 2 x Oxygesic 20 + 2 x Naloxone 10** | **1 x OXN 40/20 / 2 x Oxygesic 20+2 x Naloxone 10** | **4 x OXN 18/5/ 2 x OXN 20/10** | **4 x OXN 10/5 / 1 x OXN 40/20** | **2 x OXN 20/10 / 1 x OXN 40/20** |
|---|---|---|---|---|---|---|
| **AUCt(pg.h/mL)** | | | | | | |
| Ratio (%) | 101.0 | 98.8 | 98.6 | 102.2 | 102.4 | 100.2 |
| 90%CI | 95.6,106.8 | 93.4,104.5 | 93,3,104.3 | 96.7,108.1 | 97.0,108.2 | 94.8,105.9 |
| **AUCINF(pg.h/m L)** | | | | | | |
| Ratio (%) | 102.1 | 98.2 | 99.0 | 104.0 | 103.1 | 99.2 |
| 90%CI | 96.3,108.3 | 92.3,104.2 | 93.4, 105.0 | 97.9,110.5 | 97.3,109.3 | 93.5,105.2 |
| **Cmax (pg/mL)** | | | | | | |
| Ratio (%) | 95.4 | 96.5 | 95.1 | 98.8 | 100.3 | 101.5 |
| 90%CI | 88,5, 102,8 | 89.4, 104.1 | 88.2, 102.5 | 91.7,106.6 | 93.1,108.0 | 94.1,109.3 |
| **tmax (h)** | | | | | | |
| Difference | -0.34 | -0.16 | -0.42 | -0.18 | 0.08 | 0.26 |
| 90%CJ | -0.84,0.17 | -0.67,0.35 | -0.93,0.10 | -0.69,0.33 | -0.43,0.59 | -0.26,0.77 |
| **t1/2Z(h)** | | | | | | |
| Difference | 0.87 | 0.37 | 0.32 | 0.50 | 0.56 | 0.06 |
| 90%CI | -0.02,1.77 | -0.53,1.28 | -0.58,1.21 | -0.41,1.41 | -0.33,1.45 | -0.85,0.96 |

**Table 18. Summary of Pharmacokinetic Parameters for Naloxone by Treatment: Full Analysis Population for Pharmacokinetics.**

| **Pharmacokinetic parameter** | **4 x OXN 10/5** | **2 x OXN 20/10** | **1 x OXN 40/20** | **2 x Oxygesic 20 + 2 x Naloxone 10** |
|---|---|---|---|---|
| **AUCt(pg.h/mL)** | | | | |
| N | 24 | 23 | 23 | 23 |
| Arithmetic Mean | 0.84 | 0.89 | 0.87 | 0.97 |
| (SD) | (0.656) | (0.749) | (0.718) | (0.976) |
| Geometric Mean | 0.67 | 0.70 | 0.68 | 0.72 |
| **AUCINF(pg.h/mL)** | | | | |
| N | 2 | 6 | 0 | 1 |
| Arithmetic Mean | - | 1.64 | - | - |
| (SD) | - | (1.043) | - | - |
| Geometric Mean | - | 1.45 | - | - |
| **Cmax (pg/mL)** | | | | |
| N | 24 | 23 | 23 | 23 |
| Arithmetic Mean | 0.07 | 0.08 | 0.08 | 0.08 |
| (SD) | (0.065) | (0.106) | (0.071) | (0.101) |
| Geometric Mean | 0.06 | 0.06 | 0.06 | 0.06 |
| tmax (h) | | | | |
| N | 24 | 23 | 23 | 23 |
| Median | 4.0 | 5.0 | 2.0 | 1.0 |
| (Min,Max) | (0.5,12.0) | (0.5,24.0) | (0.5,12.0) | (0.5,24.0) |
| t1/2Z | | | | |
| N | 4 | 9 | 4 | 4 |
| Arithmetic Mean | 9.89 | 12.85 | 13,83 | 11.02 |
| (SD) | (3.137) | (11.924) | (1.879) | (1.075) |

**Table 19. Naloxone Summary of Ratios for AUCt, AUCINF. Cₘₐₓ and Differences for Tₘₐₓ and Half-Life - Full Analysis Population or Pharmacokinetics.**

| **Pharmacoki netic metric** | **4 x OXN 10/5 / 2 x Oxygesic 20 + 2 x Naloxone 10** | **2 x OXN 20/10 /2 x Oxygesic 20 + 2 x Naloxone 10** | **1 x OXN 40/20 / 2 x Oxygesic 20 + 2 x Naloxone 10** | **4 x OXN 10/5 / 2 x OXN 20/10** | **4 x OXN 10/5 / 1 x OXN 40/20** | **2 x OXN 28/10 / 1 x OXN 40/20** |
|---|---|---|---|---|---|---|
| **AUCt** | | | | | | |
| **(pg.h/mL)** | | | | | | |
| Ratio (%) | 94.2 | 99.4 | 94.1 | 4.7 | 100.1 | 105.7 |
| 90%CI | 82.0, 108.2 | 86.3, 114.5 | 81.8, 108.1 | 82.4, 108.9 | 87.3,114.9 | 92.0, 121.5 |
| **AUCINF** | | | | | | |
| **(pg.h/mL)** | | | | | | |
| Ratio (%) | - | - | - | - | - | - |
| 90%Cl | -- | -- | -- | -- | -- | -- |
| **Cmax** | | | | | | |
| **(pg/mL)** | | | | | | |
| Ratio (%) | 102.4 | 108.8 | 104.1 | 94.1 | 98.4 | 104.5 |
| 90%Cl | 88.0,119.2 | 93.1,127.0 | 89.3, 121.2 | 80.8, 109.7 | 84.6, 114.4 | 89.7,121.8 |
| **tmax (h)** | | | | | | |
| Difference | -0.71 | 0.12 | -2.03 | -0.83 | 1.32 | 2.15 |
| 90%CI | -2.96, 1.54 | -2.17,2.42 | -4.31,0.24 | -3.10, 1.44 | -0.93, 3.57 | -0.12, 4.43 |
| **t1/2Z (h)** | | | | | | |
| Difference | -3.53 | 0.79 | 2.30 | -4.35 | -5.85 | -1.51 |
| 90%CI | -12.92, 5.82 | -23.09, 24.67 | -22.06,26.67 | -28.49,19.80 | -30.48, 18.77 | -8.80,5.78 |

### 6. Data Analysis

a) Oxycodone Results
   - AUCt
      The AUCt values obtained for oxycodone were very consistent between the treatments. Each of the treatments had a mean AUCt value of between 473 ng.h/mL (4 x OXN 10/5) and 502 ng.h/mL (2 x Oxygesic 20 mg & 2 x naloxone CR 10 mg).
      In terms of AUCt, each of the fixed combination tablets provided an equivalent availability of oxycodone to the reference treatment, and to each other. All of the relative bioavailability calculations had 90% confidence intervals that were within the 80 - 125% limits of acceptability for bioequivalence.
   - t1/2Z
      The t1/2Z values obtained for oxycodone were consistent between the treatments. Each of the treatments had a mean t1/2Z value of between 4.69 h (4 x OXN 10/5), and 5.01 h (2 x Oxygesic 20 mg & 2 x naloxone CR 10 mg). There were no statistical differences between the t1 /2Z values for the treatments for any of the comparisons that were made.
   - AUCINF
      The AUCINF values obtained for oxycodone were very consistent between the treatments. Each of the treatments had a mean AUCINF value of between 475 ng.h/mL (4 x OXN 10/5) and 509 ng.h/mL (2 x Oxygesic 20 mg & 2 x naloxone CR 10 mg).
      In terms of AUCINF, each of the fixed combination tablets provided an equivalent availability of oxycodone to the reference treatment, and to each other. All of the relative bioavailability calculations had 90% confidence intervals that were within the 80 - -125% limits of acceptability for bioequivalence.
   - Cₘₐₓ
      The Cₘₐₓ values obtained for oxycodone were consistent between the fixed combination treatments, and ranged from 34.46 ng/mL (1 x OXN 40/20) to 35.73 ng/mL (2 x OXN 20/10). The mean Cₘₐₓ value for 2 x Oxygesic 20 mg & 2 x naloxone CR 10 mg was slightly higher at 40.45 ng/mL.
      The Cₘₐₓ ratios comparing the fixed combination tablets with each other ranged from 97.5% to 103.1%, and each had 90% confidence intervals within 80 - 125%. The higher mean Cₘₐₓ value for 2 x Oxygesic 20 mg & 2 x naloxone CR 10 mg meant that the Cₘₐₓ ratios comparing the fixed combination tablet with the reference product were lower, ranging from 85.8% to 88.4%. However, these Cₘₐₓ ratios were still associated with 90% confidence intervals that were within 80 - 125%.
   - tₘₐₓ
      The median tₘₐₓ values for the fixed combination tablets ranged from 3 h (1 x OXN 40/20) to 4 h (2 x OXN 20/10). The differences between these two treatments, although apparently small, was statistically significant. The median 4ₘₐₓ for 2 x Oxygesic 20 mg & 2 x naloxone CR 10 mg was 2.5 h, and there was a statistically significant differences between this reference treatment and 2 x OXN 20/10.
b) Naloxone-3-Glucuronide Results
   - AUCt
      The AUCt values obtained for naloxone-3-glucuronide were very consistent between the treatments. Each treatment had a mean AUCt value of between 520 ng.h/mL (1 x OXON 40/20) and 540 ng.h/mL (4 x OXN 10/5).
      In terms of AUCt, each of the fixed combination tablets provided an equivalent availability of naloxone-3-glucurouide to the reference treatment, and to each other. All of the relative bioavailability calculations had 90% confidence intervals that were within the 80 - 125% limits of acceptability for bioequivalence.
   - t1/2Z
      The t1/2Z values obtained for naloxone-3-glucuronide were consistent between the treatments. Each of the treatments had a mean t1/2Z value of between 7.66 h (2 x Oxygesic 20 mg & 2 x naloxone CR 10 mg) and 8.48 h (4 x OXN 10/5). There were no statistical differences between the t1/2Z values for the treatments for any of the comparisons that were made.
   - AUCINF
      The AUCINF values obtained for naloxone-3-glucuronide were very consistent between the treatments. Each of the treatments had a mean AUCINF value of between 521 ng.h/mL (2 x OXN 20/10) and 563 ng.h/mL (4 x OXN 10/5).
      In terms of AUCINF, each of the fixed combination tablets provided an equivalent availability of naloxone-3-glucuronide to the reference treatment, and to each other. All of the bioavailability calculations had 90% confidence intervals that were within the 80 - 125% limits of acceptability for bioequivalence.
   - Cₘₐₓ
      The Cₘₐₓ values obtained for naloxone-3-glucuronide were consistent between the treatments.Each of the treatments had a mean Cₘₐₓ value that range from 61.95 ng.mL (1 x OXN 40/20) to 63.62 ng.mL (2 x OXN 20/10).
      Each of the fixed combination tablets provided an equivalent naloxone-3-glucuronide Cₘₐₓ to the reference treatment, and to each other. All of the Cₘₐₓ ratio calculations had 90% confidence intervals that were within the 80 - 125% limits of acceptability for bioequivalence.
   - tₘₐₓ
      The median tₘₐₓ values for all the treatments ranged from 0.5 h (2 x OXN 20/10) to 1 h (4 x OXN 10/5, 1 x OXN 40/20 and 2 x Oxygesic 20 mg & 2 x naloxone CR 10 mg). There were no significant differences between the median tₘₐₓ values for any of the treatments.
   - Naloxone-3-glucuronide : naloxone AUCt ratios
      The mean naloxone-3-glucuronide : naloxone AUCt ratios ranged from 852.25 (2 x Oxygesic. 20 mg & 2 x naloxone CR 10 mg) to 933.46 (4 x OXN 10/5).
   - Naloxone-3-glucuronide : naloxone AUCINF ratios
      The lack of AUCINF estimates for naloxone meant that mean naloxone-3-glucuronide :
      naloxone AUCINF ratios were only able to be calculated for 2 x OXN 20/10 tablets. These provided a mean naloxone-3-glucuronide : naloxone AUCINF ratio of 414.56, based on 5 subjects' data.
c) Naloxone Results
   Naloxone concentrations were low, as was anticipated; therefore these results did not support a full pharmacokinetic assessment.
   - AUCt
      The AUCt values obtained for naloxone were consistent between the treatments. Each of the treatments had a mean AUCt value of between 0.84 ng.h/mL (2 x OXN 20/10) and 0.97 ng.h/mL (2 x Oxygesic 20 mg & 2 x naloxone CR 10 mg).
      In terms of AUCt, each of the fixed combination tablets provided an equivalent availability of naloxone to the reference treatment, and to each other. All of the bioavailability calculations had 90% confidence intervals that were within the 80 - 125% limits of acceptability for bioequivalence.
   - t1/2Z
      It was not possible to calculate t1/2Z values for naloxone for all of the subjects with confidence, because the plasma concentrations in the terminal part of the profile did not always approximate to a straight line when plotted on a semi-bgarithmic scale. The mean values were based on numbers of subjects ranging from 4 to 9.
      The mean t1/2Z values obtained for naloxone ranged from between 9.89 h (4 x OXN 10/5) to 13.83 h (1 x OXN 40/20). There were a wide range of t1/2Z values contributing to the means, however, there were no statistical differences between the t1/2Z values for the treatments for any of the comparisons that were made.
   - AUCINF
      AUCINF values were calculated for those subjects with an estimable t1/2Z value. Some of the AUCINF values were not reportable because the extrapolated portion of the AUC accounted for more than 20% of the AUCINF value. A mean AUCINF value, of 1.64 ng.h/mL, was reportable for 2 x OXN 20/10 tablets only. None of the other treatments had sufficient data to report a mean AUCINF value. There were insufficient data to make comparisons between the treatments.
   - Cₘₐₓ
      Each of the treatments had a mean Cₘₐₓ value of between 0.07 ng/mL (4 x OXN 10/5) and 0.08 ng/mL (2 x OXN 20/10, x OXN 40/20 and 2 x Oxygesic 20 mg & 2 x naloxone CR 10 mg).
      Each of the fixed combination tablets provided an equivalent naloxone Cₘₐₓ to each other. All of the Cₘₐₓ ratios comparing the fixed combination tablets had 90% confidence intervals that were within the 80 - -125% limits of acceptability for bioequivalence.
      When the fixed combination tablets were compared with the reference product, the 2 x OXN 20/10 tablets versus 2 x Oxygesic 20 mg & 2 x naloxone CR 10 mg bad a 90% confidence interval that was above the 80 - 125% limit of acceptability for bioequivalence. The remaining fixed combination tablets provided an equivalent naloxone Cₘₐₓ to the reference product.
   - tₘₐₓ
      The median tₘₐₓ values for the treatments ranged from 1 h (2 x Oxygesic 20 mg & 2 x naloxone CR 10 mg) to 5 h (2 x OXN 20/10). There were a wide range of tₘₐₓ values for each of the treatments. There were no significant differences between the median tₘₐₓ values for any of the treatments.
7. Clinical Pharmacology Discussion and Conclusions
   Low oral bioavailability prevents the complete pharmacokinetic assessment of naloxone. This was confirmed as the low plasma concentrations meant that it was not possible to estimate AUCINF values for naloxone for most of the subjects. Naloxone-3-glucuronide was present in the plasma in much higher concentrations, and AUCINF estimates were obtained for naloxone-3-glucuronide for the majority of subjects. The conclusions for the naloxone component of the fixed combination tablets were based on naloxone-3-glucuronide parameters.
   a) Oxycodone
      The mean plasma oxycodone concentration-time curves for 2 x Oxygesic 20 mg & 2 x naloxone CR 10 mg and the fixed combination tablets were almost superimposable.
      A bioequivalence assessment was made for oxycodone. Each of the bioequivalence comparisons had 90% confidence intervals that were within the limits of acceptability for bioequivalence for Frelt, FrelINF and Cₘₐₓ ratio. The oxycodone results indicate that each of the fixed combination tablet strengths were bioequivalent, both to each other and also to Oxygesic given together with naloxone CR tablet. There were no statistical differences between any of the tₘₐₓ or t1/2Z values for any of the treatments, further confirming the similarity of the products.
      The plasma oxycodone concentrations achieved after administration of the reference product were similar to dose-adjusted oxycodone concentrations seen after administration of OxyContin in a previous study. The mean Cₘₐₓ values for the fixed combination tablets were slightly lower, but when these were compared with the reference product, the Cₘₐₓ ratios had confidence intervals that were within the limits of acceptability for bioequivalence.
   b) Naloxone
      The mean plasma naloxone concentration were low, less than 0.1 ng/mL, and appeared to be biphasic, with a second peak occurring at between 8 to 16 hours.
      Even though all of the subjects did have quantifiable plasma naloxone concentrations, individual subjects' plasma naloxone concentrations were low and highly variable. The maximum observed plasma naloxone concentrations were 0.07 to 0.08 ng/mL.
      The pharmacokinetic profiles of naloxone from earlier studies were examined. On average, the mean Cₘₐₓ values from these studies, dose-adjusted to a single dose of 1 mg, ranged between 4 and 15 pg/mL, confirming that the low plasma naloxone concentrations observed here were consistent with those levels measured in earlier studies.
      A bioequivalence assessment was made for naloxone. The variability of the plasma naloxone concentrations did not allow for an estimate of AUCINF, or therefore FrelINF values. The bioavailability estimate was based on Frelt values. Each of the bioavailability comparisons had 90% confidence intervals that were within the limits of acceptability for bioequivalence. The mean Cₘₐₓ values for naloxone were comparable, and five out of the six bioavailability comparisons had 90% confidence intervals that met the criteria for bioequivalence.
      The tₘₐₓ and t1/2Z values for the treatments were variable, however there were no significant differences between any of the treatments for these two parameters.
      As expected, the levels of naloxone-3-glucuronide seen in the plasma after administration of the fixed combination tablets and Oxygesic plus naloxone, were much higher than the levels of naloxone that were achieved, resulting in naloxone-3-glucuronide : naloxone AUCt ratios of around 900. 6β-naloxol was also measured in higher quantities than naloxone, resulting in 6β-naloxol : naloxone AUCt ratios of around 22. These metabolite : parent AUCt ratios were consistent across the fixed combination tablets and the reference treatment.
   c) Naloxone-3-glucuronide
   The mean plasma naloxone-3-glucuronide levels were higher than naloxone, and it was possible to make a bioavailability assessment based on FrelINF values.
   A bioequivalence assessment was made for naloxone-3-glucuronide. Each of the bioequivalence comparisons had 90% confidence intervals that were within the limits of acceptability for bioequivalence for Frelt, FrelINF and Cₘₐₓ ratio. The naloxone-3-glucuronide results indicate that each of the fixed combination tablet strengths were bioequivalent to each other, and to Oxygesic plus naloxone. There were no statistical differences between any of the tₘₐₓ or t1/2Z values for any of the treatments, further confirming the similarity of the products.

## Claims

1. Use of an opioid agonist or a pharmaceutically acceptable salt thereof in combination with an opioid antagonist or a pharmaceutically acceptable salt thereof in the manufacture of a pharmaceutical dosage form for treating Crohn's disease and/or for treating symptoms associated with Crohn's disease in humans or animals.

2. Use according to claim 1 wherein the opioid agonist is selected from the group consisting of morphine, oxycodone, hydromorphone,oxymorphone, propoxyphene, nicomorphine, dihydrocodeine, diamorphine, papavereturn, codeine, ethylmorphine, phenylpiperidine, methadone, dextropropoxyphene, buprenorphine, pentazocin, tilidine, tramadolm, hydrocodone, and the salts thereof and wherein the opioid antagonist is selected from the group consisting of naloxone, naltrexone, nalmefene, nalorphine, nalbuphin, naloxonazinene, methylnaltrexone, ketylcyclazocine, norbinaltorphimine, naltrindol, 6-β-naloxol, 6- β-naltrexol, and salts thereof.

3. Use according to claim 1 or 2, wherein the opioid agonist and/or the opioid antagonist are present in the form of their pharmaceutically acceptable salts, preferably as the hydrochloride, sulfate, bisulfate, tatrate, nitrate, citrate, bitartrate, phosphate, malate, maleate, hydrobromide, hydroiodide, fumarate or succinate salt.

4. Use according to any of claims 1 to 3, wherein the opioid agonist or its pharamaceutically acceptable salt and the opioid antagonist or its pharmcaceutically acceptable salt are released from the dosage form in a sustained manner.

5. Use according according to claim 4, wherein the sustained release properties are essentially provided by a matrix.

6. Use according to claim 5, wherein the sustained release properties are essentially provided by a diffusion matrix.

7. Use according to claim 6, wherein said diffusion matrix comprises at least one hydrophobic polymer and/or at least one fatty alcohol.

8. Use according to claim 7, wherein said hydrophobic polymer is a hydrophobic cellulose ether and preferably ethylcellulose.

9. Use according to claim 7, wherein said fatty alcohol is lauryl alcohol, myrestyl alcohol, stearyl alcohol, cetostearyl alcohol, ceryl alcohol and/or cetyl alcohol.

10. Use according to claim 4, wherein the sustained release properties are essentially provided by a coating.

11. Use according to claim 10, wherein the coating is made from a polymer, preferably from ethylcellulose and/or an acrylic polymer resin.

12. Use according to any of claims 1 to 11, wherein the dosage form further comprises an immediate release phase of the opioid agonist or a pharmaceutically acceptable salt thereof.

13. Use according to any of claims 1 to 12, wherein the dosage form is a tablet, a capsule, a dragée, a granulate and/or a powder.

14. Use according to any of claims 1 to 13, wherein the dosage form provides a therapeutic efficacy for at least 8 hours, preferably for at least 12 hours or at least 24 hours.

15. Use according to any of claims 1 to 14, wherein the dosage form comprises oxycodone or a pharmaceutically accepptable salt thereof and naloxone or a pharamaceutically acceptable salt thereof.

16. Use according to claim 15, wherein the pharmaceutically acceptable salt thereof is the hydrochloride salt of oxycdone and naloxone.

17. Use according to claim 15 or 16, wherein the dosage form comprises between 5 to 160 mg of oxycodone or a pharmaceutically acceptable salt thereof and 2.5 to 40 mg of naloxone or a pharmaceutically acceptable salt thereof.

18. Use according to any of claims 15 to 17, wherein the dosage form comprises oxycodone and naloxone or their pharmaceutically acceptable salts in a weight ratio of 5:1, 4:1, 3:1 and preferably of 2:1 oxycodone: naloxone.

19. Use according to any of claims 15 to 18, wherein the dosage form comprises 10 mg of oxycodone or a pharmaceutically acceptable salt thereof and 5 mg of naloxone or a pharmaceutically acceptable salt thereof, 20 mg of oxycodone or a pharmaceutically acceptable salt thereof and 10 mg of naloxone or a pharmaceutically acceptable salt thereof or 40 mg of oxycodone or a pharmaceutically acceptable salt thereof and 20 mg of naloxone or a pharmaceutically acceptable salt thereof.

20. Use according to any of claims 15 to 19 wherein the dosage form releases oxycodone or a phamaceutcially acceptable salt thereof and naloxone or a pharmaceutically acceptable salt thereof by the following in vitro dissolution rates when measured according to the European Pharmacopeia paddle test at 50 rpm in 900 ml 0.1 N HCl pH 1.2 using UV detection at 270 nm:
10 - 30 % by weight of oxycodone or said salt thereof at 15 min,
40 - 65 % by weight of oxycodone or said salt thereof at 2 h,
≥ 80% by weight of oxycodone or said salt thereof at 10 h,
10 - 30 % by weight of naloxone or said salt thereof at 15 min,
40 - 65 % by weight of naloxone or said salt thereof at 2 h,
≥ 80% by weight of naloxone or said salt thereof at 10 h.

21. Use according to any of claims 15 to 20 wherein the dosage form releases oxycodone or a pharmaceutcially acceptable salt thereof and naloxone or a pharmaceutically acceptable salt thereof by the following in vitro dissolution rates when measured according to the European Pharmacopeia paddle test at 50 rpm in 900 ml 0.1 N HCl pH 1.2 using UV detection at 270 nm:
10 - 30 % by weight of oxycodone or said salt thereof at 15 min,
30-50 % by weight of oxycodone or said salt thereof at I h,
40 - 65 % by weight of oxycodone or said salt thereof at 2 h,
60 - 85 % by weight of oxycodone or said salt thereof at 4 h,
70 - 95 % by weight of oxycodone or said salt thereof at 7 h,
≥ 80% by weight of oxycodone or said salt thereof at 10 h,
10 - 30 % by weight of naloxone or said salt thereof at 15 min,
30 - 50 % by weight of naloxone or said salt thereof at 1 h,
45 - 65 % by weight of naloxone or said salt thereof at 2 h,
60 - 85 % by weight of naloxone or said salt thereof at 4 h,
70 - 95 % by weight of naloxone or said salt thereof at 7 h, and
≥ 80% by weight of naloxone or said salt thereof at 10 h.

22. Use according to any of claims 15 to 21, wherein the dosage form provides a mean tₘₐₓ for oxycodone at about 1 to about 17 hours, at about 2 to about 15 hours, at about 3 to about 8 hours or at about 4 to about 5 hours after single dose administration to healthy human subjects.

23. Use according to any of claims 15 to 22, wherein the dosage form provides a mean AUCt value for oxycodone of about 100 ng•h/mL to about 600 ng•h/mL, about 400 ng•h/mL to about 550 ng•h/mL, or about 450 to about 510 ng•h/mL after single dose administration to healthy human subjects.

24. Use according to any of claims 15 to 22, wherein the dosage form provides a mean Cₘₐₓ for oxycodone of about 5 ng/mL to about 50 ng/mL, about 30 ng/mL to about 40 ng/mL or about 35 ng/mL after single dose administration to healthy human subjects.

25. Use according to any of the preceeding claims of an opioid agonist or a pharmaceutically acceptable salt thereof in combination with an opioid antagonist or a pharmaceutically acceptable salt thereof in the manufacture of a pharmaceutical dosage form for treating pain as a major symptom associated with Crohn's disease in humans or animals.
